# EUROPEAN PATENT SPECIFICATION

(11) **EP 4 277 747 B1**
(45) Date of publication and mention of the grant of the patent: **19.03.2025**
(21) Application number: 22700115.3
(22) Date of filing: 12.01.2022
(51) Int. Cl.: B01L 3/00, C12Q 1/6813

(54) **ANALYSIS OF CELLS AND/OR ORGANELLES IN HYDROGEL CAGES**
ANALYSE VON ZELLEN UND/ODER ORGANELLEN IN HYDROGELKÄFIGEN
ANALYSE DE CELLULES ET/OU D'ORGANITES DANS DES CAGES D'HYDROGEL

(30) Priority: 12.01.2021 EP 21305027
(43) Date of publication of application: 22.11.2023
(73) Proprietor: Paris Sciences et Lettres, 75006 Paris (FR); Ecole Supérieure de Physique et de Chimie Industrielles de la Ville de Paris, 75005 Paris (FR); Centre National de la Recherche Scientifique, 75016 Paris (FR); Microfactory, 75005 Paris (FR); SORBONNE UNIVERSITE, 75006 Paris (FR)
(72) Inventor: GEISLER, Hubert, 75004 PARIS (FR); TABELING, Patrick, 75005 PARIS (FR); GRIFFITHS, Andrew, 75006 PARIS (FR); CHIRON, Stéphane, 69008 LYON (FR); MONTI, Fabrice, 91160 SAULX LES CHARTREUX (FR); BLIVET-BAILLY, Gaël, 75005 PARIS (FR); ABDORAHIM, Marjan, 92160 ANTONY (FR); TRAN-AMARELIS, Yvette, 75013 PARIS (FR); NGHE, Phillipe, 94160 SAINT-MANDE (FR)
(74) Representative: Novagraaf Group
(86) International application number: PCT/EP2022/050518
(87) International publication number: WO 2022/152739

(56) References cited:
- WO-A1-2020/123309
- WO-A2-2013/067525
- DE-U1- 202020 004 663

## Description

The present invention relates to a device for biological analyses, notably for analysis of cells and/or organelles. The invention also relates to a process for the manufacture of said device and its different applications for biological analyses.

Single cell multi-omics analysis is a growing field. The 2018 market was $300M, and it is predicted to be $1.6B in 2022 (Biolnformatics, LLC). Single-cell analysis is an indispensable tool to overcome the cellular heterogeneity of healthy and diseased tissues. Indeed, the analysis of a set of cells cannot reveal the heterogeneity of its composition. For example, tumors typically comprise multiple types of tumor cells: a cancerous tissue from a breast cancer patient may, for example, comprise both HER2 positive and HER2 negative cells, the former responding and the latter not responding to therapy with the therapeutic antibody trastuzumab (Herceptin^{®}). In addition, tumors typically also contain a wide range of stromal and tumor-infiltrating immune cells. An analysis of the whole tumor would never reveal this granularity.

"Omic" analyses (genomic, epigenomic and transcriptomic analyses) using barcoded next-generation sequencing are now possible at high-throughput at the single-cell scale. Single-cells are isolated in compartments and nucleic acids released from the cells are labeled with nucleic acid sequence barcodes that are unique for each compartment, and hence for each cell. The labelled nucleic acids are pooled and analyzed by next-generation sequencing. Subsequent bioinformatics analysis allows reads to be assigned to single cells as reads from the same cell carry the same barcode. Likewise, phenotypic analyses are also now possible at high-throughput at the single-cell scale, using microscopy, flow-cytometry and mass-cytometry. CITE-seq additionally allows analysis of a sub-fraction of the proteome, displayed on the cell surface, to be coupled to transcriptomic analysis via barcoded next-generation sequencing. However, high-throughput single-cell omics data from barcoded next-generation sequencing has not been mapped onto phenotypic data from imaging.

There is today a strong need, expressed by the research and clinical community, for a better understanding of complex and heterogeneous biological systems, requiring the acquisition of both phenotypic (protein expression, protein secretion, glycosylation, post-translational modification, metabolism, cell morphology, cellular response to external stimuli...) and omics information for a single cell, and this for thousands of cells of a biological sample.

To answer some research or clinical questions, it may be necessary to only analyze a sub-population of interest. Reducing the number of cells to be sequenced makes it possible to focus on rare events, complex sets of cell populations or to provide tools for competitive theranostic purposes. While sub-populations of cells can be pre-enriched, for example by fluorescence-activated cell sorting (FACS) or using magnetic beads prior to single-cell sequencing, there is currently no integrated system that allows cells to be monitored and selectively single-cell sequencing of a sub-population of cells with a specific phenotype or phenotypes. Furthermore, pre-enrichment using FACS or magnetic beads is poorly adapted to the analysis of small numbers of cells (for example from tumor biopsies) and does not allow classification of cells based on temporal monitoring of cells, or based on detection of secreted molecules (for example antibodies and cytokines).

### State of the art

The current systems allow to isolate cells in compartments, release the nucleic acids from the cells and label them with nucleic acid sequence barcodes that are unique for each compartment, and hence for each cell, prior to next-generation sequencing. However, there are currently no systems that allow analysis of large numbers of cells (>10,000) and that allow to directly couple the phenotype of the cell, determined by optical imaging, to its -omic characteristics (genome, epigenome, transcriptome, ...), nor to select cells of interest and sequence them individually.

Existing technologies only allow to:
1. Perform barcoded single-cell sequencing of large numbers of cells (>10,000) cells using droplet microfluidics, in which single cells are compartmentalized in droplets together with single beads carrying nucleic acid sequence barcodes that are unique for each bead, without imaging, fluorescence measurement or sorting of droplets;
2. Perform single-cell analysis of small numbers of cell (≤800) cells using valve microfluidics, in which single cells are compartmentalized in microfabricated chambers separated by pneumatic valves, with temporal imaging by microscopy, followed by single-cell sequencing, without sorting or selective sequencing of cells with specific phenotypes (Fluidigm C1, Fluidigm Polaris);
3. Single-cell sequencing of a small number of cells (<2,000) in which single cells are compartmentalized in microplate wells for single-cell sequencing without imaging, fluorescence measurement or sorting of cells.

When using droplet microfluidics, valve microfluidics or microplate well, sub-populations of cells can be pre-enriched prior to compartmentalization, based on fluorescent-labelling of small panels of cellular markers (<50), typically cell-surface markers, and fluorescence-activated cell-sorting (FACS), or using magnetic beads labeled with ligands against cell-surface markers. However, there is no one-to-one mapping of fluorescence data onto sequencing data for each cell analyzed. However, when using microplate wells, individual cells can be sorted into individual wells by FACS, prior to single-cell sequencing, with one-to-one mapping of the fluorescence profile of the cell to the omics data. Furthermore, other systems such as the CellenONE stystem and the Berkeley Lights optofluidics system allow imaging of individual cells followed by selective dispensing of individual cells into microplate wells.

However, no technology currently available allows phenotypic monitoring after the compartmentalization of >10,000 cells, with the possibility of selecting a sub-population to be analyzed by sequencing.

In particular, no technology currently available allows coupling omic analyses (genomic, epigenomic, transcriptomic and proteomic analyses) to analyses of phenotype by optical imaging.

D'Eramo L.; et al., Microsystems & Nanoengineering, 2018, 4, 17069, doi:10.1038 teaches a microfluidic device comprising microfluidic actuators based on temperature-responsive hydrogels. However, the disclosed device does not comprise an array of cages with walls comprised of temperature-responsive hydrogels in combination with a nucleic acid array, the nucleic acid array comprising a plurality of nucleic acids grafted onto one of the substrates, wherein each nucleic acid comprises a barcode that encodes the position of the nucleic acid on the substrate. Therefore, the disclosed device cannot be used for barcoded single-cell sequencing.

WO2020123309 discloses a microfluidic device comprising a substrate including spatially barcoded nucleic acid probes and a sample, wherein the sample can be a flexible array, wherein the array is immobilized on a hydrogel.

WO2013067525 discloses a microlfuidic device including a base with a pattern of actuatable hydrogel structures, which can swell to an upright position or contract to a lying position, depending on stimulus.

### Advantages of the invention

The microfluidic device according to the invention allows the analysis of single, or small groups of cells, notably at high throughput (more than 10,000 cells) and under very competitive cost conditions, with one-to-one mapping of phenotypic data from optical imaging onto single-cell omics data from barcoded next-generation sequencing. It further allows selective sequencing of sub-populations of cells based on phenotypic data from optical imaging.

In particular, the microfluidic device according to the invention allows isolating single cells or organelles in microcompartments (cages) made of activatable hydrogel allowing visualization, labeling and biological analysis of the cells and the genetic material found in each isolated cell. The use of an expandable gel gives the ability to introduce and isolate, on demand, a population of cells or organelles in microcompartments so that a single cell or organelle is arranged in a cage while providing a biological seal between cages. The use of barcoded nucleic acids, fixed inside the cages, allows labeling ("barcoding") of any nucleic acids released from or associated with the cells or organelles, in the cages.

This new technique allows high-throughput analysis and monitoring of the phenotype of the single cells by optical imaging, prior to cell lysis and sequencing, which allows one-to-one mapping of cellular phenotype (protein expression, protein secretion, glycosylation, post-translational modification, metabolism, cell morphology, cellular response to external stimuli...) onto single-cell omic data from barcoded sequencing. However, the system also has a number of other advantageous features, including:
- Imaging allows quality control of the experiment, providing information about the fraction of cages loaded with cells, the type of cells, the viability of cells and the efficiency of cell lysis;
- Imaging allows exclusion of sequencing data from cages with the wrong number of cells during bioinformatic analysis, for example, only data from cages with single cells can be retained;
- In one embodiment of the invention, only a sub-population of cells, identified based on phenotypic data from optical imaging, are analyzed by sequencing;
- In contrast to systems based on compartmentalization in droplets, reagents can be added or removed at different steps during the analyses, thanks to the capacity to open, partially open, or close the cages, which induces a level of flexibility in the protocols that is currently not possible with competing technologies;
- Analyses are user-friendly and fully automatable

The coupling of single-cell omic and phenotypic analyses by imaging allows a better understanding of bio-systems complexity. The invention would also permit more precise diagnosis and stratification of patients and better theranostic precision.

### Summary of the invention

The invention relates to a microfluidic device comprising:
- a first wall comprising a first substrate on which a plurality of closed patterns is grafted,
- a second wall, facing the first wall, comprising a second substrate,
- a plurality of nucleic acids grafted either on the first substrate or on the second substrate, wherein each nucleic acid comprises a barcode that encodes the position of the nucleic acid on said first or second substrate,

wherein at least the plurality of closed patterns or the second substrate is made of an actuatable hydrogel which is swellable between a retracted state and a swollen state in which the closed patterns and the second substrate come into contact.

The invention further relates to a method of manufacture of a device according to the invention, said method comprising:
1) Providing a first substrate,
2) Grafting on the surface of said first substrate a plurality of closed patterns,
3) Providing a second substrate,
4) Grafting a plurality of nucleic acids either on the surface of the first substrate, or on the surface of the second substrate, wherein each nucleic acid comprises a barcode that encodes the position of the nucleic acid on said first or second substrate;
5) Positioning the first substrate and the second substrate by placing the closed patterns and the nucleic acids between the first substrate and the second substrate,
6) Bonding the first and the second substrates.

The invention further relates to a method of performing analysis of cells or organelles comprising:
a) Providing a microfluidic device according to the invention, and a preparation of cells or organelles;
b) Optionally, associating all or part of the cells or organelles with a common labeling nucleic-acid sequence or with a plurality of different labeling nucleic-acid sequences;
c) Injecting in the microfluidic device the cells or organelles in suspension under conditions in which the hydrogel is in retracted state;
d) Modifying the conditions to actuate the hydrogel into swollen state, thereby trapping cells or organelles in a cage formed by the first and second walls of the microfluidic device, and the closed pattern of hydrogel in swollen state;
e) Optionally analyzing captured cells or organelles and/or molecules they secrete, using optical imaging;
f) Optionally releasing in the cage, the grafted nucleic acids from the surface of the first or second substrate of the microfluidic device;
g) Optionally, lysing trapped cells or organelles, thereby releasing cellular or organellar nucleic acids in the cages;
h) Associating the barcode of the nucleic acids with either the released cellular or organellar nucleic acids and/or labeling nucleic-acid sequence(s) thereby forming barcoded nucleic acids;
i) Modifying the conditions to actuate the hydrogel into the retracted state;
j) Releasing the grafted nucleic acids from the first or second substrate of the microfluidic device, if not released in f);
k) Recovering and sequencing the barcoded nucleic acids.

### Detailed Description of the invention

The invention firstly relates to a microfluidic device comprising:
- a first wall comprising a first substrate on which a plurality of closed patterns is grafted,
- a second wall, facing the first wall, comprising a second substrate,
- a plurality of nucleic acids grafted either on the first substrate or on the second substrate, each nucleic acid comprising a barcode that encodes the position of the nucleic acid on said first or second substrate,
wherein at least the plurality of closed patterns or the second substrate is made of an actuatable hydrogel which is swellable between a retracted state and a swollen state in which the closed patterns and the second substrate come into contact.

The invention also relates to a process for the manufacture of the microfluidic device as defined above.

The invention further concerns a method of performing a biological analysis, notably a cell or organelle analysis, by using a microfluidic device as defined above.

All the embodiments and the preferential features detailed hereafter apply independently to all the objects of the invention, notably to the device, the process of manufacture and different applications thereof.

### The device

In the swollen state, the closed patterns and the second substrate of the device are in contact. The device thus comprises a plurality of cages, each cage being delimited by a lateral wall made of the closed patterns and by end walls constituted of the first and the second substrates.

In the retracted state, the closed patterns and the second substrate are no more in contact. A gap between the closed patterns and the second substrate allows fluids and cells freely circulating inside the device.

Between the retracted and the swollen states, the device according to the invention goes through a multitude of intermediary states wherein the actuatable hydrogel is only partially swollen. In these configurations, a gap between the closed patterns and the second substrate still exists. However, the height of the gap is sufficiently reduced with respect to the retracted state so that cells, captured in the cages, are retained in the cages. These intermediary configurations may typically be used to allow a selective passage of fluids but not cells.

Each closed pattern thus defines a trapping site for cells wherein closure and opening are initiated by an external stimulus. In a preferred embodiment, the external stimulus is a change in pH, in light intensity, in temperature or in electrical current intensity. In a highly preferred embodiment, the external stimulus is a change in temperature.

The microfluidic device comprises at least 2 closed patterns. Preferably, the microfluidic device comprises a large number of closed patterns, typically 100, 1,000, 10,000, 100,000 ...

The first wall and the second wall are made of a rigid material that is capable of resisting temperature fluctuations ranging from -20°C to 100°C.According to a first embodiment, the walls (first and/or second walls) are made of a unique and homogenous material. The wall thus consists of the substrates.

According to a second embodiment, the walls further comprise a support material on which is fixed/coated the substrate. Typically, the wall consists of a support material made of glass or polydimethylsiloxane, covered with a substrate layer.

The device according to the invention may equivalently comprises two monolayer walls, two multilayer walls or one monolayer wall and one multilayer wall.

The first substrate is typically made of a material chosen from: silicon, quartz, glass, polydimethylsiloxane, thermoplastics such as cyclic olefin copolymers and polycarbonates, preferably from glass and polydimethylsiloxane.

Preferably, the first substrate is made of polydimethylsiloxane.

According to an embodiment, at least part of the surface of the first substrate is structured and/or functionalized.

By "structured", we mean in the context of the invention that the surface of the substrate is irregular. The surface of the substrate may be porous or microscopically structured. In particular, it can comprise microscopic streaks, pillars, etc...

The structuration of the substrate may be performed according to any known process. Mention may for example be made of standard soft-lithography techniques which are well documented.

By "functionalized", we mean in the context of the invention that the fixation of chemical functional groups on the surface of the substrate. Typically, the surface of the first substrate is functionalized with chemical groups chosen from hydroxide groups, silanol groups and mixtures thereof, preferably silanol groups.

The structuration and/or the functionalization of the substrate(s) permit to facilitate the grafting of the closed patterns and/or of the nucleic acids on their surface.

According to a preferential embodiment, the first wall is made of a structured and/or functionalized polydimethylsiloxane substrate, preferably of a structured and functionalized polydimethylsiloxane substrate.

The closed patterns may have a large variety of shape. Preferably, the closed patterns are rectangular, square, circular or hexagonal.

Preferably, the closed patterns are covalently grafted to the first substrate.

According to a particular embodiment, the second substrate is made of the hydrogel and the closed patterns are made of a non-swellable material.

Preferably, according to this particular embodiment, the second wall comprises a non-swellable support material on which is deposited the swellable hydrogel.

The non-swellable support material may be structured and/or functionalized. Structuration and/or functionalization of the non-swellable material are made by analogy with what has been said above in the context of the first substrate.

Thus, according to this embodiment, the closed patterns are non-swellable and it is the swelling of the second substrate that permits the closure of the cages.

Preferably, according to this embodiment, the closed patterns are made of a material chosen from silicon, quartz, glass, polydimethylsiloxane, thermoplastic materials such as cyclic olefin copolymers and polycarbonates, preferably from glass or polydimethylsiloxane.

Preferably, the closed patterns have a height ranging from 0.1 µm to 100 µm, preferably from 1 µm to 30 µm.

Preferably, the walls of closed patterns have a thickness ranging from 0.1 to 500 µm, preferably from 1 µm to 20 µm.

Advantageously, according to this embodiment, the second substrate has a thickness, measured in the swollen state into contact with the closed patterns, ranging from 1 µm to 500 µm, preferably from 1 µm to 100 µm.

Advantageously, still according to this embodiment, the second substrate comprising the hydrogel has a thickness, measured in the dry state, ranging from 0.5 µm to 150 µm, preferably from 0.5 µm to 50 µm.

According to a preferred embodiment, the closed patterns are made of the hydrogel and the second substrate is made of a non-swellable material.

Thus, according to this embodiment, the second substrate is non-swellable and the closed patterns swell to close the cages.

Preferably, according to this preferred embodiment, the second substrate is made of a material chosen from: silicon, quartz, glass, polydimethylsiloxane, thermoplastics such as cyclic olefin copolymers and polycarbonates, preferably from glass or polydimethylsiloxane. Advantageously, the hydrogel patterns have a height, measured in the swollen state when the hydrogel patterns are in contact with the second wall, ranging from 0.1 µm to 500 µm, preferably from 1 µm to 250 µm, more preferably from 1 µm to 100 µm.

Advantageously, the hydrogel pattern has a height, measured in the dry state, ranging from 0.1 µm to 150 µm, preferably from 0.5 µm to 100 µm, more preferably from 0.5 µm to 50 µm.

Preferably, the walls of the hydrogel patterns have a resolution, measured in the swollen state when the hydrogel patterns are in contact with the second wall, ranging from 0.1 µm to 100 µm, preferably from 1 µm to 10 µm.

Preferably, the walls of the hydrogel patterns have a resolution, measured in the dry state, ranging from 0.1 µm to 100 µm, preferably from 0.5 µm to 5 µm.

By "hydrogel", we refer in the context of the invention to a polymeric material that, when placed in an aqueous medium, notably water, and in certain physical and/or chemical conditions, swells by absorbing and retaining said aqueous phase.

By "hydrogel", we refer in the context of a gel comprising a polymer matrix forming a three-dimensional network which is capable of swelling in the presence water, under specific physico-chemical conditions. In particular, the swelling of the hydrogel may be initiated and/or modulated by a thermal, optical, chemical or electrical stimulus.

For example, the swelling (or the deflation) of the hydrogel may be initiated and/or modulated by a change in temperature, in pressure or in the pH value of the medium wherein it is placed.

Preferably, the hydrogel is a temperature-responsive swellable hydrogel. By "temperature-responsive swellable hydrogel", we refer in the context of the invention to a hydrogel which swelling or deflation is induced by varying the temperature. A temperature-responsive swellable hydrogel typically exhibits a drastic change of water-solubility with temperature.

In a specific range of temperature, the hydrogel is water-soluble and absorb large quantities of water.

Reversely, by changing the temperature of the medium, the hydrogel becomes no more water-soluble. The hydrogel then releases water and deflates.

By "swollen state", we refer in the context of the invention to a state of the hydrogel wherein the closed patterns and the second substrate are in contact such that the device comprises a plurality of hermetically sealed cages.

By "retracted state", we refer in the context of the invention to a state of the hydrogel wherein the closed patterns and the second substrate are not in contact: a gap between the closed patterns and the second substrate exists and permits a free circulation of fluids and cells inside the microfluidic device. The "retracted state" differs from the "dry state" define below in that the hydrogel is not completely free from water. In the retracted state, the hydrogel is still at least partially hydrated.

By "dry state", we refer in the context of the invention to a state wherein the hydrogel is almost completely free from water. Typically, the hydrogel is in the dry state during the manufacture of the microfluidic device, notably during the grafting of the hydrogel patterns of during the coating of the second wall with the hydrogel substrate.

The temperature at which the water-solubility properties of the hydrogel drastically change is designated as the critical solution temperature (CST).

Preferably, the hydrogel has a critical solution temperature (CST) ranging from 4°C to 98°C, more preferably from 20°C to 50°C, even more preferably from 25°C to 40°C.

According to a first variant, the critical solution temperature (CST) of the hydrogel is a lower critical solution temperature (LCST). At a temperature superior to the LSCT, the hydrogel is in the retracted state and at a temperature inferior to the LCST, the hydrogel is in the swollen state.

According to a second variant, the critical solution temperature (CST) of the hydrogel is an upper critical solution temperature (UCST). At a temperature superior to the UCST, the hydrogel is in the swollen state and at a temperature inferior to the USCT, the hydrogel is in the retracted state.

The polymer constituting the polymer matrix of the hydrogel is typically chosen from homopolymers, copolymers and terpolymers of acrylics, alkyl (meth)acrylates, alkyl (meth)acrylamides, oligoethylene (meth)acrylates, sulfobetaines (meth)acrylates and N-acryloyl glycinamide, preferably chosen from homopolymers copolymers and terpolymers of alkyl (meth)acrylamides and any mixtures thereof, more preferably the hydrogel comprises poly(N-lsopropylacrylamide).

The polymer may be chosen from LCST polymers, UCST polymers and mixtures thereof.

By analogy with what has been said above in the context of the hydrogel:
- the expression "LCST polymer" designates a thermo-responsive polymer having a lower critical solution temperature, and
- the expression "UCST polymer" designates a thermo-responsive polymer having an upper critical solution temperature.

The overall behavior of the hydrogel (UCST and/or LCST behavior) depends on the nature and on the amount of the different polymers present in the hydrogel.

When the polymer is chosen from UCST polymers, it is preferably chosen from homopolymers, copolymers and terpolymers of acrylics, alkyl (meth)acrylates, alkyl (meth)acrylamides, oligoethylene (meth)acrylates, sulfobetaines (meth)acrylates, N-acryloyl glycinamide and mixtures thereof.

Preferably, the UCST polymer is a terpolymer of methacrylamide, acrylamide and allylmethacrylate.

When the polymer is chosen from LCST polymers, it is preferably chosen from homopolymers, copolymers and terpolymers of acrylics, alkyl (meth)acrylates, alkyl (meth)acrylamides, oligoethylene (meth)acrylates and mixtures thereof, more preferably from homopolymers, copolymers and terpolymers of alkyl (meth)acrylamides, event more preferably the LCST polymer is poly(N-lsopropylacrylamide).

Preferably, the LCST polymer is poly(N-lsopropylacrylamide).

Advantageously, the polymer comprises, preferably consists of, one or several UCST or LCST polymers.

Advantageously, the microfluidic device further comprises at least one inlet and at least one outlet permitting respectively the introduction and the removal of reactants into the device.

Preferably, heating means are integrated in the device according to the invention.

According to an embodiment, each cage comprises independent heating means. This embodiment is particularly advantageous in that it permits to open and close each cage independently.

For example, local heating means may consist of nanoparticles, which heat up when irradiated with light (Plasmonic effect). The nanoparticles may for example be deposited between the hydrogel and the wall on which it is coated or dispersed in the polymer matrix of the hydrogel. Preferably, the nanoparticles are chosen from metal nanoparticles and plasmonic nanoparticles, preferably comprises gold, graphene, silver, copper and titanium nitride.

In another example, local heating is performed using microresistors; for example microresistors comprising chromium/gold bilayer or TiO₂ structures.

The microfluidic device further comprises a plurality of nucleic acids grafted either on the first substrate or on the second substrate, wherein each nucleic acid comprises a sequence barcode that encodes the position of the nucleic acid on said first or second substrate.

Advantageously, the nucleic acids are grafted so as to be placed inside the cages, when the hydrogel is in the swollen state.

More advantageously, the nucleic acids are grafted either on the first substrate, inside the closed patterns or on the second substrate, opposite the closed patterns.

Preferably, when the closed patterns are made of the hydrogel, the nucleic acids are grafted on the surface of the second substrate. Preferably, when the second substrate is made of the hydrogel, the nucleic acids are grafted on the surface of the first substrate.

The grafted nucleic acids are RNA or DNA, preferably DNA. The grafted nucleic acids can be single-stranded, double-stranded or partially double-stranded.

The grafted nucleic acids are preferably 60 to 100 nucleotide long.

The grafted nucleic acid may be attached to the substrate at the 3'-end or 5'-end, either directly, or by a linker.

According to an embodiment, grafted nucleic acids sharing the same barcode have a plurality of sequences. According to another embodiment, grafted nucleic acids sharing the same barcode have a same sequence.

According to an embodiment, all or part of the grafted nucleic acids are hybridized to another nucleic acid or a plurality of nucleic acids and form a partly or fully double stranded DNA, double stranded DNA/RNA, or double stranded RNA.

According to an embodiment, the grafted nucleic acids comprise one or any combinations of the following sequences:
1) a restriction site or a photocleavable site for nucleic acid release,
2) a sequence complementary to an amplification primer for further amplification,
3) a T7 RNA polymerase promoter sequence for further in vitro transcription (IVT),
4) a hybridization site for nucleic acid labeling, a ligation site for nucleic acid labeling or a recombination site for nucleic acid labeling, and
5) a sequence of randomized nucleotide residues that function as a unique molecular identifier (UMI).

Preferably, the grafted nucleic acids comprise at least i) a sequence barcode that encodes the position of the nucleic acid on said first or second substrate, and ii) a restriction site or a photocleavable site, and optionally further iii) a primer sequence, and/or T7 sequence and/or hybridization, ligation or recombination site.

According to an embodiment, the grafted nucleic acids of the microfluidic device comprise a constant sequence, i.e. a sequence which is present in all grafted nucleic acids. Grafted nucleic acids of the microfluidic device may be hybridized to a DNA comprising a sequence complementary to all or part of the constant sequence of the grafted nucleic acids. One, or a plurality of different DNA comprising a sequence complementary to all or part of the constant sequence, may be hybridized to the grafted nucleic acids.

The microfluidic device according to the invention may further comprise structures capable of capturing a cell or an organelle. Such structures are typically chosen from publications as such as Vigneswaran N. et al, 2017, Microfluidic hydrodynamic trapping for single cell analysis: mechanisms, methods and applications, Anal. Methods,9, 3751-3772*.*

Preferably, the structures capable of capturing a cell or an organelle are localized either on the first substrate, inside the closed patterns or on the second substrate, opposite the closed patterns

Preferably, each cage comprises at least one structure capable of capturing a cell or an organelle.

According to a particular embodiment, a plurality of ligands is grafted, directly or indirectly, covalently or non-covalently, on the first substrate (14) and/or on the second substrate (20), opposite the closed patterns.

Advantageously, the ligands are grafted so as to be placed inside the cages, when the hydrogel is in the swollen state.

In particular, when grafted on the first substrate (14), the ligands are typically grafted inside the closed patterns (16).

Alternatively, when grafted on the second substrate (20), the ligands are facing the closed patterns.

The ligands may all be grafted on the same substrate. Alternatively, some of the ligands are grafted on the first substrate (14) and the others are grafted on the second substrate (20).

Preferably, when grafted directly on the first (14) or second (20) substrate, the plurality of ligands is covalently grafted on the first (14) or second (20) substrate.

According to a more specific embodiment, the plurality of ligands is grafted indirectly: the plurality of ligands is grafted to an intermediate structure, said intermediate structure being directly grafted on the first (14) or second (20) substrate. Thus, according to this specific embodiment, there is no direct bonding between the plurality of ligands and the substrates (14, 20).

Preferably, when grafted indirectly on the first (14) or second (20) substrate, the plurality of ligands is grafted non-covalently on the first (14) or second (20) substrate.

According to first example, the plurality of ligands is conjugated with a nucleic acid and is associated by hybridization to at least part of the grafted nucleic acids (22).

According to another example, the plurality of ligands are non-covalently grafted to an adhesion coating previously coated on the first (14) or second (20) substrate. As adhesion coatings, mentions may notably be made to streptadivin coatings.

In these embodiments, preferably, each ligand is independently chosen from the group consisting of antibodies, fragments of antibody, lectins, and aptamers.

The ligands are usually selected to bind one or more analyte(s) secreted or released by lysis of the cell(s) or organelles trapped in the cage formed by the first (14) and second (20) walls of the microfluidic device (10), and the closed pattern (16) of hydrogel in swollen state.

### The process of manufacture

The invention also relates to a method for the manufacture of a microfluidic device as defined above.

Preferably, the method of manufacture comprises the following steps:
1) Providing a first substrate,
2) Grafting on the surface of said first substrate a plurality of closed patterns,
3) Providing a second substrate,
4) Grafting a plurality of nucleic acids either on the surface of the first substrate, or on the surface of the second substrate, wherein each nucleic acid comprises a barcode that encodes the position of the nucleic acid on said first or second substrate;
5) Positioning the first substrate and the second substrate by placing the closed patterns and the nucleic acids between the first substrate and the second substrate,
6) Bonding the first and the second substrates.

The grafting of the closed patterns may be performed according to any known process.

When the closed patterns are made of a non-swellable material, the grafting of the closed patterns is typically performed by soft-lithography techniques.

According to a particular embodiment, the first substrate and the closed patterns are prepared together in a one and unique step.

When the closed patterns are made of the hydrogel, the grafting of the closed pattern is typically performed by photopatterning, preferably under UV (Ultraviolet) radiation. Photopatterning methods consists in the surface-grafting of the polymer matrix of hydrogel on the first substrate, and simultaneously by the crosslinking of the polymer matrix of the hydrogel.

Preferably, the polymers are covalently crosslinked.

More preferably, the crosslinking of the polymer is made in presence of a crosslinking agent chosen from dithiol molecules such as for example dithioerythriol.

The patterning of the hydrogel is typically performed by standard photolithographic techniques or with a direct LASER writing equipment.

These techniques are notably disclosed in Chollet, B., D'Eramo, L., Martwong, E., Li, M., Macron, J., Mai, T.Q., Tabeling, P. and Tran, Y., 2016. Tailoring patterns of surface-attached multiresponsive polymer networks. ACS applied materials & interfaces, 8(37), pp.24870-24879.

The grafting of the nucleic acids is typically performed by spotting or in-situ light directed synthesis, respectively detailed in DeRisi, J. et al. Use of a cDNA microarray to analyse gene expression. Nat. Genet 14, 457-460 (1996) and in Fodor, S. P. et al. Lightdirected, spatially addressable parallel chemical synthesis. Science (80-.). 251, 767-773 (1991).

Advantageously, during step 5), the first and the second substrates are positioned in a way permitting the nucleic acids to be inside the cages, when the hydrogel is in the swollen state.

More advantageously, the nucleic acids are grafted either on the first substrate, inside the closed patterns or on the second substrate, opposite the closed patterns.

The bonding step may be performed according to any known process.

According to a first embodiment, the bonding step is performed by oxygen plasma treatment.

Preferably, the oxygen plasma treatment is made at room temperature, typically at a temperature ranging from 5°C to 50°C, more preferably from 10°C to 40°C, even more preferably from 15°C to 30°C.

Preferably, the duration of the oxygen plasma treatment ranges from 10 s to 2 min, more preferably from 30 s to 1 min.

Preferably, according to this first embodiment, the process further comprises, before step 6), a preparation step of deposition on the nucleic acids of a mask capable of protecting the nucleic acids during exposure to the oxygen plasma.

The mask is typically made of an adhesive tape, preferably made of a material chosen from plastic film, paper, cloth, foam or foil coated with an adhesive.

The mask is finally removed after the plasma treatment, typically by peeling.

According to a second embodiment, the bonding step is performed by the application of a pressure on the surface of the device.

Preferably, according to this embodiment, the pressure on the surface of the device is performed by applying a negative pressure into an external microfluidic channel surrounding the main design.

According to third embodiment, the bonding step is performed by using of a crosslinkable composition comprising at least one polymer and optionally at least one crosslinking agent.

According to this third embodiment, the bonding step is performed as follows:
a) bringing together the first and the second walls,
b) depositing between the two walls a layer of a composition comprising at least one polymer and at least one crosslinking agent in order to fill the gap between the first wall and the second wall, and
c) crosslinking, preferably self-crosslinking, of the at least one polymer.

Preferably, the polymer is chosen among polyepoxides

The process may further comprise:
- between steps 1) and 2), an intermediary step of structuring and/or functionalizing the surface of the first substrate, and/or
- between steps 3) and 4), an intermediary step of structuring and/or functionalizing the surface of the second substrate.

When the substrate is made of a hydrogel, the functionalization of the substrate may typically be performed by following the protocol described in Chollet, B. D'eramo, L., Martwong, E., Li, M., Macron, J., Mai, T.Q., Tabeling, P. and Tran, Y., 2016. Tailoring patterns of surface-attached multiresponsive polymer networks. ACS applied materials & interfaces, 8(37), pp.24870-24879.

If the structures aren't made of hydrogel, then functionalization may typically be performed following the protocol detailed in Beal, John H L et al. "A rapid, inexpensive surface treatment for enhanced functionality of polydimethylsiloxane microfluidic channels." Biomicrofluidics vol. 6,3 36503. 30 Jul. 2012

When the substrate is made of a hydrogel, the structuration of the substrate may typically be performed by following the protocol described in Chollet, B. D'eramo, L., Martwong, E., Li, M., Macron, J., Mai, T.Q., Tabeling, P. and Tran, Y., 2016. Tailoring patterns of surface-attached multiresponsive polymer networks. ACS applied materials & interfaces, 8(37), pp.24870-24879.

When the substrate is made of a non-swellable material, the structuration of the substrate may typically be performed following standard lithography protocols, notably standard photolithography protocols.

According to a particular embodiment, the process may further comprise, before the deposition the hydrogel material, an additional step consisting of the deposition on the surface of the substrate of a nanoparticle layer, preferably a patterned chromium/gold bilayer.

The deposition of the patterned layer may for example be performed by standard photolithography.

According to a particular embodiment, the method further comprises at least one of the following steps:
a) grafting (directly) a plurality of ligands on the surface of the first substrate (14) and/or on the surface of the second substrate (20), and/or
b) grafting (indirectly) a plurality of ligands on the surface of the first substrate (14) and/or on the surface of the second substrate (20).

Step a) defined above may be performed at any time of the manufacturing method defined above. In particular, step a) may be performed before or after the grafting of the closed patterns (16), before or after the grafting of the nucleic acids (22).

According to a first embodiment, the indirect grafting of ligands is performed by associating to the plurality of grafted nucleic acids (22) a plurality of ligands by hybridization, said plurality of ligands being conjugated with a nucleic acid having complementarity with at least a part of the grafted nucleic acids (22).

According to this first embodiment, step b) is preferably performed after the grafting of the nucleic acids (22). Step b) can be performed until the conditions are modified to actuate the hydrogel into swollen state, thereby trapping cells or organelles in a cage formed by the first (14) and second (20) walls of the microfluidic device (10), and the closed pattern (16) of hydrogel in swollen state.

According to a second embodiment, the indirect grafting of the ligands comprises i) coating an adhesion coating on at least part of the surface of the first (14) and/or second (20) substrate, and ii) grafting the ligands to said adhesion coating.

Step i) can be performed before or after the grafting of the closed patterns (16), before or after the grafting of the nucleic acids (22).

Step ii) is preferably performed after the deposition of the adhesion coating. Step ii) can be performed until the conditions are modified to actuate the hydrogel into swollen state, thereby trapping cells or organelles in a cage formed by the first (14) and second (20) walls of the microfluidic device (10), and the closed pattern (16) of hydrogel in swollen state.

The method for the manufacture of a microfluidic device further comprises one or more of the following steps:
1) Hybridizing a DNA comprising a sequence complementary to all or part of a constant sequence present in grafted nucleic acids, in particular present in all or part of the grafted nucleic acids ; in particular, one or a plurality of different DNA comprising a sequence complementary to all or part of the constant sequence may be used;
2) Extending the hybridizing DNA by polymerisation (e.g. using Maxima, SuperScript RT, Phusion or Q5 polymerase);
3) Ligating the grafted nucleic acids, in particular grafted DNA, with a or another DNA sequence; and/or
4) Releasing all or part of the grafted nucleic acid, possibly previously modified by hybridization, extension or ligation according to 1), 2) or 3), from the surface of the first or second substrate, by cleavage (for instance by photocleavage or cleavage catalyzed by an endonuclease).

In some embodiments, the Hybridizing DNA forms together with the grafted nucleic acid, a double stranded DNA containing a restriction site for an endonuclease.

The process may also comprise a further step of fixing structures capable of capturing a cell or an organelle.

This supplemental step is typically realized by standard photolithography.

### Applications

The microfluidic device of the invention can be used in methods of analysis of cells or cell organelles, with the possibility of combining phenotypic information from optical imaging and -omics information for a single cell or organelle, or for e.g. two or more cells in interaction, and this for thousands of cells simultaneously.

The method of performing analysis of cell or organelles comprises:
a) Providing a microfluidic device according to the invention and a preparation of cells or organelles;
a) Optionally, associating all or part of the cells or organelles with a common labeling nucleic acid sequence or with a plurality of different labeling nucleic acid sequences;
b) Injecting in the microfluidic device the cells or organelles in suspension under conditions in which the hydrogel is in retracted state;
c) Modifying the conditions to actuate the hydrogel into swollen state, thereby trapping cells or organelles in a cage formed by the first and second walls of the microfluidic device, and the closed pattern of hydrogel in swollen state;
d) Optionally analyzing captured cells or organelles and/or molecules they secrete, using optical imaging;
e) Optionally releasing in the cage, the grafted nucleic acids from the surface of the first or second substrate of the microfluidic device;
f) Optionally, lysing trapped cells or organelles, thereby releasing cellular or organellar nucleic acids in the cages;
g) Associating the barcode of the nucleic acids with either the released cellular or organellar nucleic acids and/or labeling nucleic acid sequence(s) thereby forming barcoded nucleic acids;
h) Modifying the conditions to actuate the hydrogel into the retracted state;
i) Releasing the grafted nucleic acids from the first or second substrate of the microfluidic device, if not released in f);
j) Recovering and sequencing the barcoded nucleic acids; and
k) Optionally mapping the barcoded sequencing data onto the data from optical imaging obtained in e).

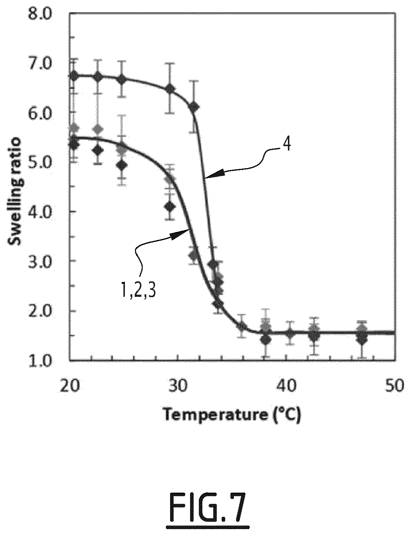

At step c), injecting in the microfluidic device the cells or organelles in suspension under conditions in which the hydrogel is in retracted state is typically performed by setting the temperature, pressure or pH - depending on the nature of the actuatable hydrogel - so that the hydrogel is in retracted state. For instance, if the microfluidic device comprises a lower critical solution temperature (LCST) temperature-responsive hydrogel, the temperature of the microfluidic device is raised above the lower critical solution temperature (LCST) to retract the hydrogel. For a temperature-responsive hydrogel comprising or consisting of poly(N-isopropylacrylamide (PNIPAM), the hydrogel is fully expanded at ≤28°C, fully retracted at ≥36°C, and partially expanded between these temperatures (see Figure 7 with temperature response), allowing cages to be fully open at 37°C for cell or organelle loading (D'Eramo et al., Microsystems & Nanoengineering (2018) 4, 17069). For instance, if the microfluidic device comprises an upper critical solution temperature (UCST) temperature-responsive hydrogel, the temperature of the microfluidic device is decreased below the upper critical solution temperature (UCST) to retract the hydrogel. For a temperature-responsive hydrogel comprising or consisting of P(MA-AM-AMA) the hydrogel is fully retracted at ≤10°C, fully expanded at ≥50°C, and partially expanded between these temperatures, allowing cages to be fully open at 10°C for cell or organelle loading. According to an embodiment, at step d), single cells or single organelles are trapped in the cages. According to another embodiment two (or more) interacting cells are trapped in the cages, for example plasma cell and reporter cell; cytotoxic T cell (or CAR T cell) and target cell (e.g. tumor cell); T cell and antigen-presenting cell.

To actuate the hydrogel into swollen state, the temperature, pressure or pH - depending on the nature of the actuatable hydrogel - is modified so that the hydrogel swells and comes into contact with the second substrate. For instance, if the microfluidic device comprises a lower critical solution temperature (LCST) temperature-responsive hydrogel, the temperature of the microfluidic device is reduced below the lower critical solution temperature (LCST) to swell the hydrogel. For a temperature-responsive hydrogel comprising or consisting of poly(N-isopropylacrylamide) (PNIPAM), the temperature can typically be set at ≤28°C, where the hydrogel is fully expanded (D'Eramo et al., Microsystems & Nanoengineering (2018) 4, 17069). For instance, if the microfluidic device comprises an upper critical solution temperature (UCST) temperature-responsive hydrogel, the temperature of the microfluidic device is raised above the upper critical solution temperature (UCST) to expand the hydrogel. For a temperature-responsive hydrogel comprising or consisting of P(MA-AM-AMA) the hydrogel is fully expanded at ≥50°C.

The method may further comprise, between steps d) and h), changing surrounding conditions of the cells or organelles. Changing surrounding conditions includes circulating in the microfluidic device an aqueous phase containing, e.g. salts, detergents, proteins, and/or nucleic acid sequences. Changing surrounding conditions includes exchanging molecules, such as salts, that pass through the hydrogel of closed cages, by fully opening cages in the case that cages also comprise structures capable of capturing a cell or an organelle, or partially opening the cages.

According to an embodiment, the method further comprises, for example after step d) and before step e), but not necessarily:
e1) Binding of an analyte or of analytes secreted or released by the captured cells or organelles to ligands grafted directly or indirectly to the surface of the first substrate (14) and/or on the surface of the second substrate (20);
e2) Detecting the analyte or analytes bound to the grafted ligand by binding with a labeled second ligand or labeled ligands that is/are specific for the bound analyte or analytes.

According to a first embodiment, at step e2, detecting is performed directly with a second ligand or ligands fluorescently labeled.

According to a second embodiment, at step e2, detecting is performed indirectly, with a second ligand or ligands labeled with a ligand identification nucleic acid that is specific for the analyte or analytes that is(are) bound to the grafted ligand, wherein the sequence of said ligand identification nucleic acid allows identification of the ligand and the analyte or analytes bound to the grafted ligand.

According to this second embodiment, the method may further comprise amplifying the sequence of said ligand identification nucleic acid. Amplification preferably consists of a linear amplification, more preferably by using at least one polymerase and at least one restriction or nicking enzyme.

According to this second embodiment of the method, in step h), the method may further comprise associating the barcode of the nucleic acids (22) with the ligand identification nucleic acid, thereby forming barcoded nucleic acids.

According to an embodiment, the common labeling DNA sequence or plurality of different labeling DNA sequences provided at step b) are used for DNA-toolbox reactions (or dynamic DNA reaction network) for phenotype sorting of cells or organelles, thereby actuating the release of the grafted nucleic acids in step f) or j). The principles of DNA-toolbox reactions are described for instance in the international patent applications WO2017141068 and WO2017141067.

According to an embodiment, at step g), trapped cells or organelles are lysed by osmotic shock. This may be readily implemented by the skilled person by circulating in the microfluidic device a hypo- or hyper-osmotic aqueous phase. The cages may be retained closed for this operation.

According to an embodiment, step h) comprises hybridizing the nucleic acids comprising barcodes, which may be still grafted the surface of the first or second substrate of the microfluidic device or released from the surface of the first or second substrate of the microfluidic device, by complementarity to the released cellular or organellar nucleic acids and/or to labeling nucleic acid sequence(s). In particular, where the nucleic acids comprising a barcode are DNA, step h) [or the method between steps i) and j)] may additionally comprise extending the DNA comprising a barcode hybridized to the released cellular or organellar nucleic acids (or labeling nucleic acid sequence(s)) using a DNA polymerase to create the complementary strand of the released cellular or organellar nucleic acids (or labeling nucleic acid sequence(s)) which comprises a barcode. The nucleic acids may comprise, for example, a 3'-region of sequence oligo d(T) or oligo d(T)VN, for hybridization to the poly(A) tail of mRNA (for mRNA sequencing), a 3'-region of sequence complementary to that of a specific RNA (for targeted RNA sequencing) or DNA (for targeted DNA sequencing), a 3'-region of random sequence, for example d(N)6 (for RNA sequencing or DNA sequencing), a 3'-region with three ribo(G) nucleotides for reverse transcriptase template switching (for RNA sequencing), or a 3'-region complementary to a nucleotide sequence introduced by recombination, for example after "tagmentation" catalyzed by Tn5 transposase. The latter can be used, for example, for genomic DNA sequencing, or epigenomic analysis of DNA methylation (using Methyl-seq or bisulfite sequencing) or chromatin structure (using transposase-accessible chromatin with sequencing, ATAC-Seq), or for RNA sequencing after tagmentation of RNA-DNA duplexes formed after first strand cDNA synthesis or double-stranded DNA formed after first and second strand cDNA synthesis on RNA released by the cells or organelles.

According to another embodiment, the nucleic acids comprising a barcode are DNA, and may be wholly or partly double-stranded, and step h) comprises ligating the DNA comprising a barcode to DNA released by the cells or organelles. For example, the barcodes may be ligated to genomic DNA, for example after restriction digestion (for genomic DNA sequencing or analysis of DNA methylation), or after digestion with micrococcal nuclease (for metagenomic analysis using MNase-seq or ChIP-seq).

According to still another embodiment, the nucleic acids comprising a barcode are DNA, and may be wholly or partly double-stranded, and step h) comprises recombining the DNA comprising barcode with DNA released by the cells or organelles. For example, the barcodes may be recombined with genomic DNA for genomic DNA sequencing, or epigenomic analysis of DNA methylation (using Methyl-seq or bisulfite sequencing) or chromatin structure (using transposase-accessible chromatin with sequencing, ATAC-Seq). Alternatively, the nucleic acids comprising a barcode recombine with RNA-DNA duplexes formed after first strand cDNA synthesis on RNA released by the cells or organelles, or recombining with double-stranded DNA formed after first and second strand cDNA synthesis on RNA released by the cells or organelles (for RNA sequencing). In a preferred embodiment the oligonucleotide comprises a Mosaic End (ME) sequence which recombines with DNA catalyzed by Tn5 transposase.

According to an embodiment, the method further comprises between steps d) and h), releasing a the nucleic acids comprising barcodes upon the presence of a cellular or organellar material (e.g. a surface molecule, a secreted molecule, or a lysis product) in the cages, e.g. by a proximity ligation assay, or proximity extension assay

The invention is further illustrated by the following figures and examples.

### FIGURES

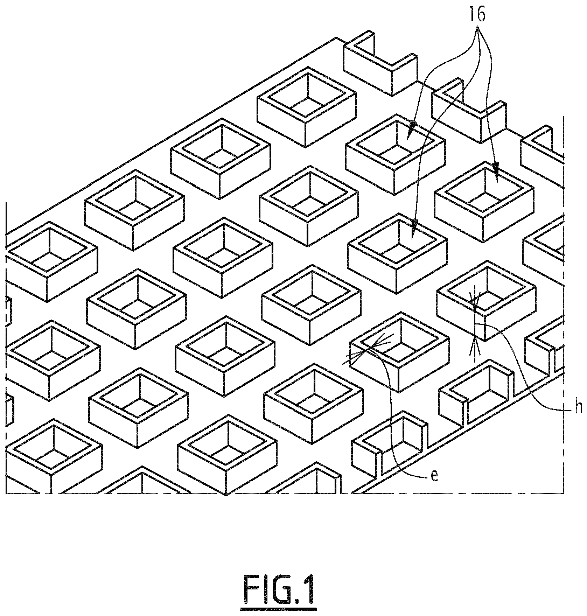

Figure 1 is a schematic representation of the closed patterns of a device according to the invention.

The microfluidic device 10 according to the invention comprises a plurality of closed patterns 16 arranged in the form of a table. The closed patterns 16 represented are in the form of squares but may equivalently in the form of rectangles, circles or even hexagons. The closed patterns 16 have a thickness e and a height h.

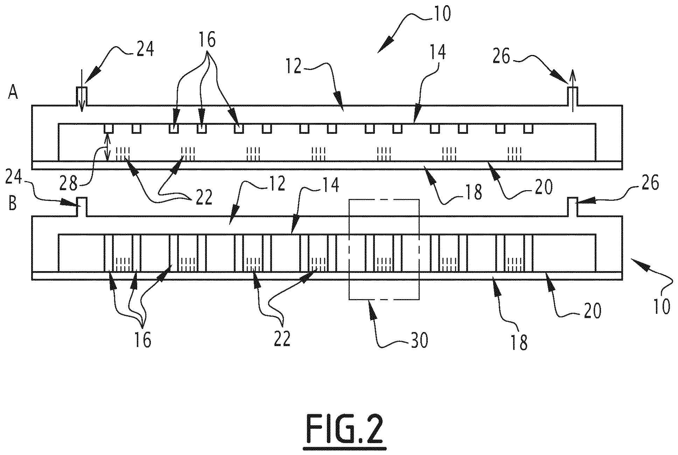

Figure 2 is a schematic representation of a microfluidic device according to a first embodiment of the invention wherein the patterns are made of an actuatable hydrogel.

The microfluidic device 10 comprises a first wall 12 comprising a first substrate 14 on which a plurality of closed patterns 16 is grafted. A second wall 18, facing the first wall 12, comprises a second substrate 20. A plurality of nucleic acids 22 are grafted on the second substrate 20. The closed patterns 16 are made of an actuatable hydrogel which is swellable between a retracted state and a swollen state in which the closed patterns 16 and the second substrate 20 come into contact. The microfluidic device 10 further comprises an inlet 24 and an outlet 26 permitting respectively the introduction and the removal of reactants into the device 10.

On scheme A, the closed patterns 16 are in the retracted state. A gap 28 between the closed patterns 14 and the second substrate 20 allows fluids and cells present inside the device freely circulating inside the device 10.

Placed in specific physico-chemical conditions, the closed patterns 16 begin to absorb water and swell. The closed patterns 16 thus elongates until contacting the second substrate 20.

On scheme B, the closed patterns 16 are in the swollen state, in contact with the second substrate 20. The device 10 thus comprises a plurality of cages 30, each cage 30 being delimited by a lateral wall made of one of the closed patterns 16 and by end walls constituted of a portion of the first 14 and second 20 substrates.

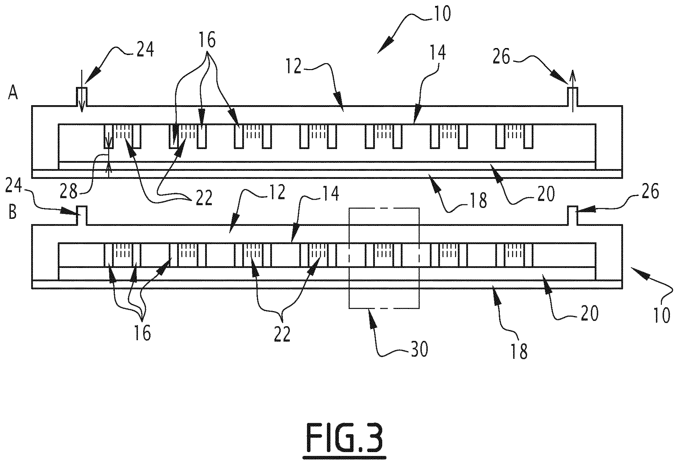

Figure 3 is a schematic representation of a microfluidic device according to a second embodiment of the invention wherein the second substrate is made of an actuatable hydrogel.

The microfluidic device 10 comprises a first wall 12 comprising a first substrate 14 on which a plurality of closed patterns 16 is grafted. A second wall 18, facing the first wall 12, comprises a second substrate 20. A plurality of nucleic acids 22 are grafted on the first substrate 14. The second substrate 20 is made of an actuatable hydrogel which is swellable between a retraced state and a swollen state in which the closed patterns 16 and the second substrate 20 come into contact. The microfluidic device 10 further comprises an inlet 24 and an outlet 26 permitting respectively the introduction and the removal of reactants into the device 10.

On scheme A, the second substrate 20 is in the retraced state. A gap 28 between the closed patterns 14 and the second substrate 20 allows fluids and cells present inside the device freely circulating inside the device 10.

Placed in specific physico-chemical conditions, the second substrate 20 begins to absorb water and swells. The thickness of the second substrate 20 thus increases until contacting the closed patterns 16.

On scheme B, the second substrate 20 is in the swollen state, in contact with the closed patterns 16. The device 10 thus comprises a plurality of cages 30, each cage 30 being delimited by a lateral wall made of one of the closed patterns 16 and by end walls constituted of a portion of the first 14 and second 20 substrates.

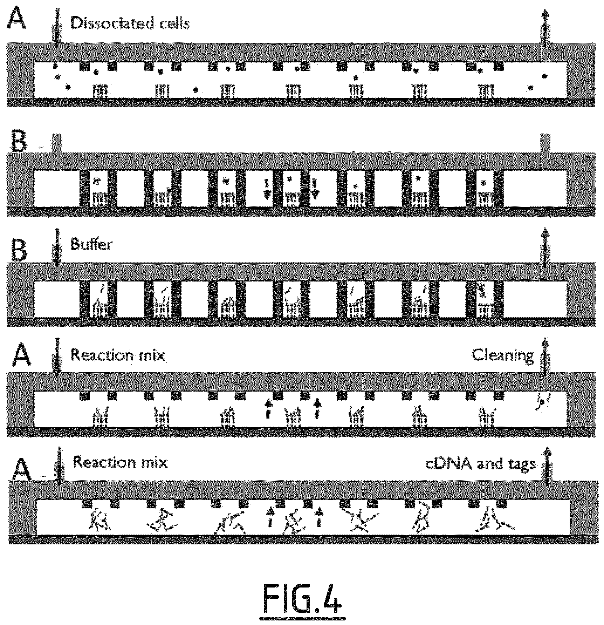

Figure 4 is a schematic representation of the main stages of the method of performing analysis of cells or organelles according to the invention. Stages are identified in ascending order from top to bottom.

Stage 1: Dissociated cells are injected into the microfluidic device at a temperature A, allowing the cages to be in the retracted state.

Stage 2: Cages are swollen by changing the temperature of the microfluidic device to temperature B allowing the capture of the cells. The cells are then lysed by changing the surrounding buffer, for example, using a low salt buffer. Untrapped cells are washed away.

Stage 3: Still at temperature B, the buffer is changed again to allow nucleic acid hybridization on the grafted oligonucleotides at the bottom of each cage.

Stage 4: Cages are retracted by changing the temperature of the microfluidic device to temperature A. This allows the injection and the incubation of a reaction mix in order to associate the barcode specific of each cage, present on the grafted oligonucleotides, to the hybridized nucleic acids released from the cell.

Stage 5: Still at temperature A, a new reaction mix is injected in order to release the grafted or hybridized barcoded cDNA and/or tags from the microfluidic device. The recovered sample is then purified, amplified and sequenced.

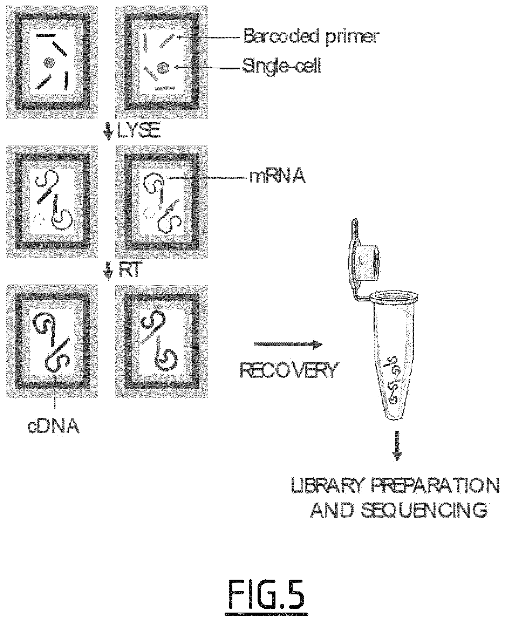

Figure 5 is a schematic representation of the succession of operations carried out in an exemplary method of performing analysis of cells or organelles according to the invention, based on transcriptome analysis. The grey rectangles represent the hydrogel cages, containing cells (circle) and capture primers (bars). The process includes, from top to bottom, a - Cages closed after capture of the cells; b - Lysis of cells by osmotic shock and hybridization of mRNA on markers in cages; c - Opening of the cages, RT (for the case of the transcriptome); d -cDNA cleavage and recovery.

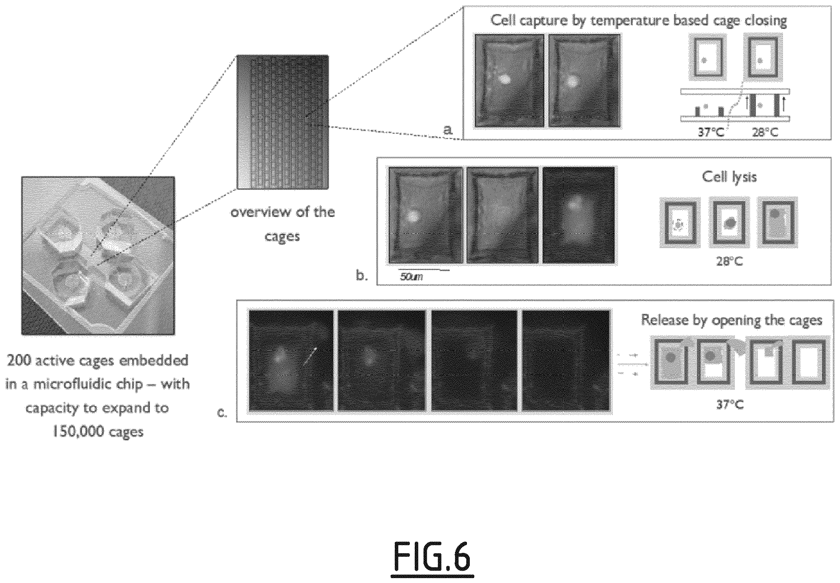

Figure 6 represents a chip including 200 cages. A zoom on the cages shows, from top to bottom, the capture (right line up), the lysis by osmotic shock, induced by the circulation of distilled water between the cages (second line), and the release of the cellular material. lysed (last line).

Figure 7 represents the swelling ratio of the PNIPAM hydrogel synthetized in Example 1, as a function of temperature, and in 4 distinct aqueous solutions. Curve 1 is has been obtained in pure water, curve 2 in a phosphate saline buffer, curve 3 at pH = 2 and curve 4 at pH = 9.

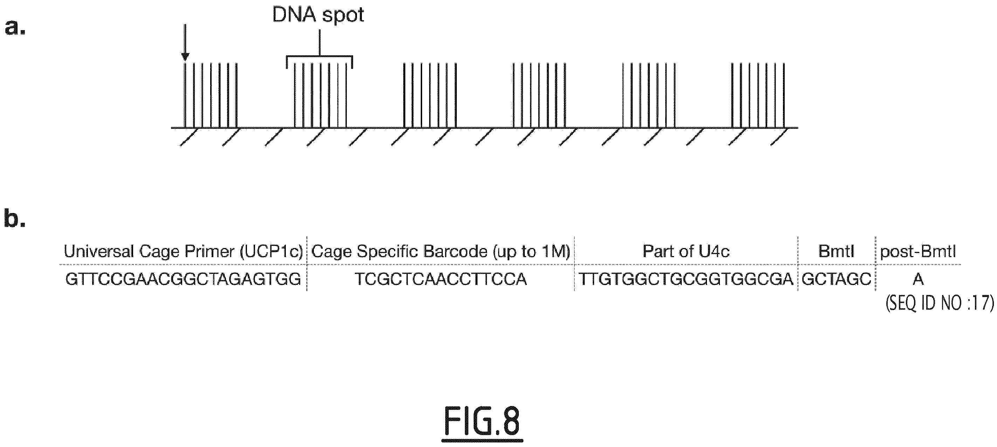

Figure 8 represents the micro array structure of the DNA strands in the microfluidic device of example 1. Figure 8a represents a section of the micro array included in the microfluidic device of example 1. The arrow indicates the position of a DNA strand. Each DNA spot is made up of a set of DNA strands. Figure 8b represents an example of composition of the DNA strands composing the DNA spots of the DNA array included in the microfluidic device of example 1. All DNA strands of a DNA spots are identical. Between each DNA spot, DNA strands differs by their spot specific barcode. Up to 1 million spot specific barcodes has been designed.

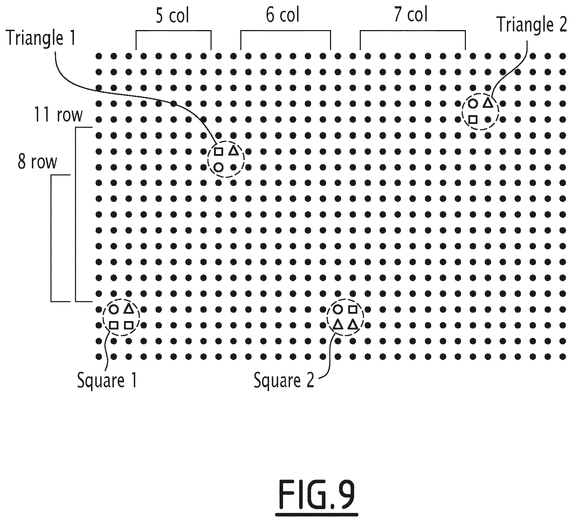

Figure 9 is a partial representation of the localization grid based on fluorescent tags. Shapes and colors define a unique location.

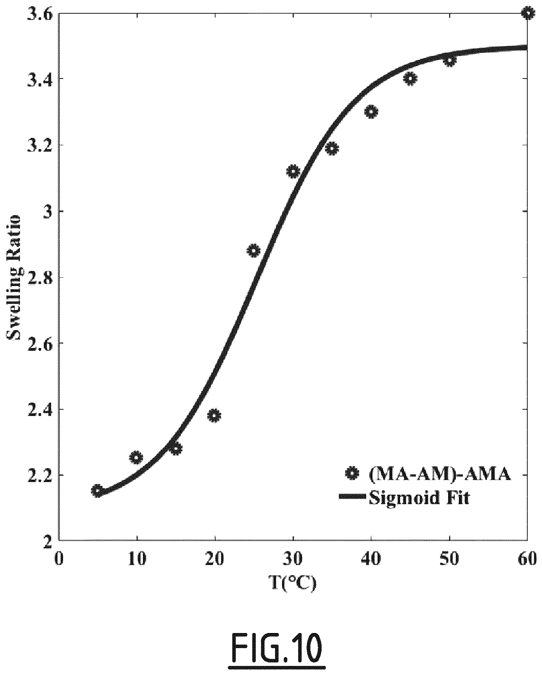

Figure 10 represents the swelling ratio of the poly(methacrylamide-acrylamiode-allylmethacrylate) hydrogel synthetized in Example 3, as a function of temperature, in a phosphate saline buffer.

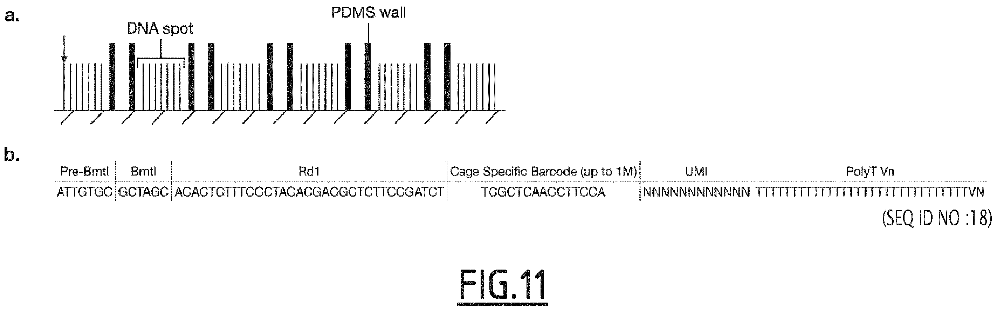

Figure 11 represents the structure of DNA microwells in the device used in example 4. Figure 11a represents a section of the micro array included in the microfluidic device of example 4. The arrow indicates the position of a DNA strand. Each DNA spot is made up of a set of DNA strands. The vertical black bars represent the wall made of PDMS that constitute cage walls. Figure 11b represents an example of composition of the DNA strands composing the DNA spots of the DNA array included in the microfluidic device of example 4 All DNA strands of a DNA spots are identical. Between each DNA spot, DNA strands differs by their spot specific barcode. Up to 1 million spot specific barcodes has been designed.

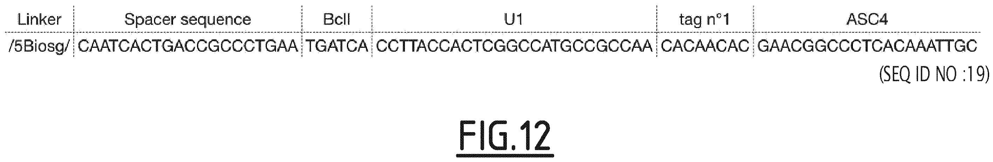

Figure 12 represents the composition of a biotinylated oligo used to label specifically cellular sub-population in example 5. Cells are labeled with sub-population specific antibody carrying antibody specific barcode. The antibody is coupled to a streptavidin (e.g. using Streptavidin Conjugation Kit - Lightning-Link from abcam - ab102921). Prior to cell labeling, a biotin conjugated oligo, as described in this figure, is added to the antibody solution in a 12:1 relation.

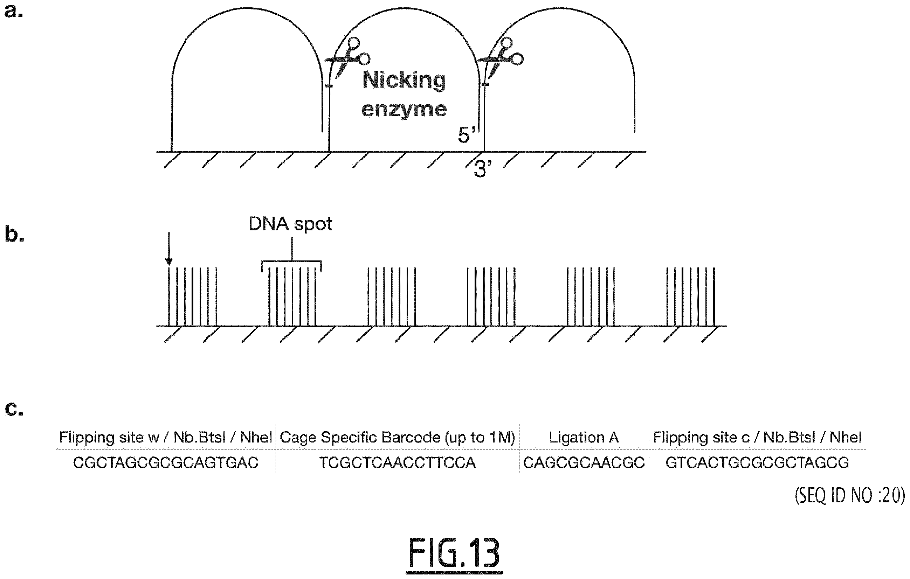

Figure 13 represents the micro array structure of the DNA strands in the microfluidic device of example 6. Figure 13a is a schematic representation of the DNA array preparation of example 6. The procedure involves the reversal of the oligonucleotides within each DNA spot of the DNA array by hybridization of each grafted strand on the neighboring grafted strand followed by the cutting of each grafted strands with a nicking restriction enzyme, in the 5' of the hybridization site. Figure 13b represents a section of the micro array included in the microfluidic device of example 6. The arrow indicates the position of a DNA strand. Each DNA spot is made up of a set of DNA strands. The vertical black bars represent the wall made of PDMS that constitute cage walls. Panel c. represents an example of composition of the DNA strands composing the DNA spots of the DNA array included in the microfluidic device of example 6 All DNA strands of a DNA spots are identical. Between each DNA spot, DNA strands differs by their spot specific barcode. Up to 1 million spot specific barcodes has been designed.

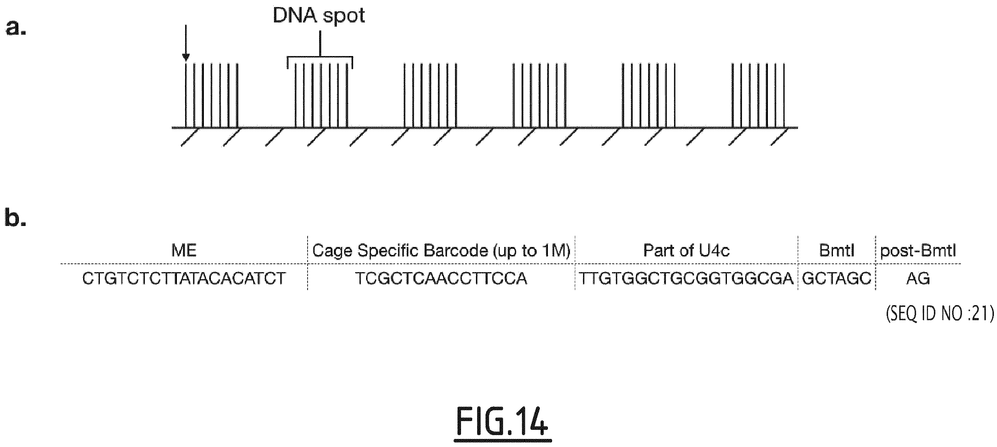

Figure 14 represents the micro array structure of the DNA strands in the microfluidic device of example 7. Figure 14a represents a section of the micro array included in the microfluidic device of example 7. The arrow indicates the position of a DNA strand. Each DNA spot is made up of a set of DNA strands. Figure 14b represents an example of composition of the DNA strands composing the DNA spots of the DNA array included in the microfluidic device of example 7 All DNA strands of a DNA spots are identical. Between each DNA spot, DNA strands differs by their spot specific barcode. Up to 1 million spot specific barcodes has been designed.

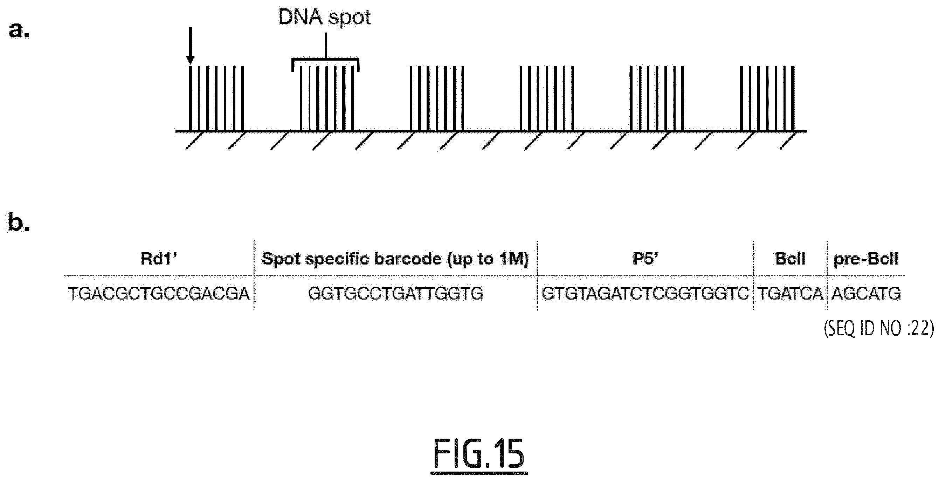

Figure 15 represents the micro array structure of the DNA strands in the microfluidic device of example 8.Figure 15a represents a section of the micro array included in the microfluidic device of example 8. The arrow indicates the position of a DNA strand. Each DNA spot is made up of a set of DNA strands. Figure 15b represents an example of composition of the DNA strands composing the DNA spots of the DNA array included in the microfluidic device of example 8. All DNA strands of a DNA spots are identical. Between each DNA spot, DNA strands differs by their spot specific barcode. Up to 1 million spot specific barcodes has been designed.

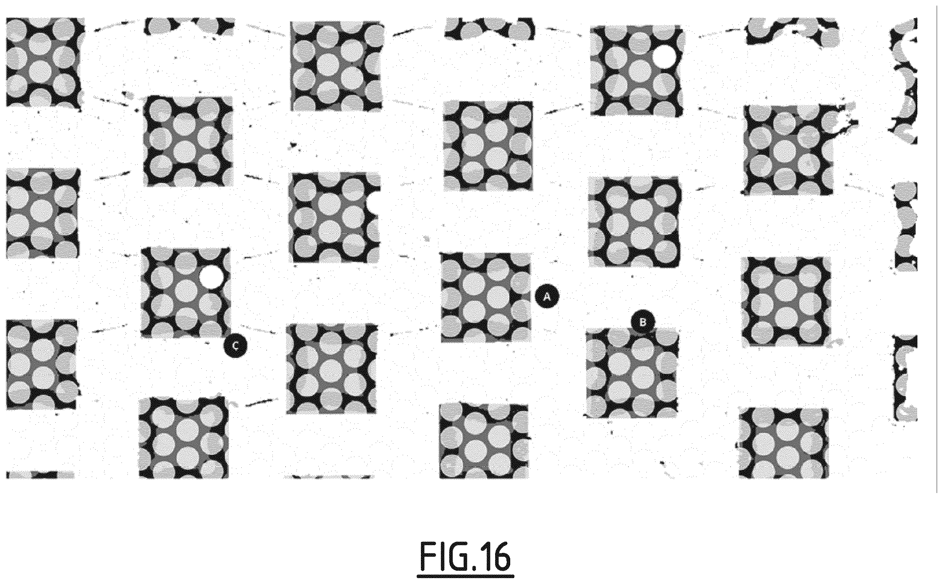

Figure 16 is an informatic identification of the DNA array spots and cage in a part of the microfluidic device of example 8. The identification is based on the superposition of a brightfield image and a fluorescent image (excitation at 650 nm and emission at 670 nm) of the microfluidic device. The brightfield image allows to identify the cages. The fluorescent image, represented by the black circles A, B and C, makes it possible to identify certain spots on which fluorescent DNA probes have been previously hybridized. The relative position of the black circle A, B and C define a unique location on the chip, allowing to map the original organization of the DNA array on the superposed images. Finally, thanks to this identification, we can associate spot specific barcodes with each cage.

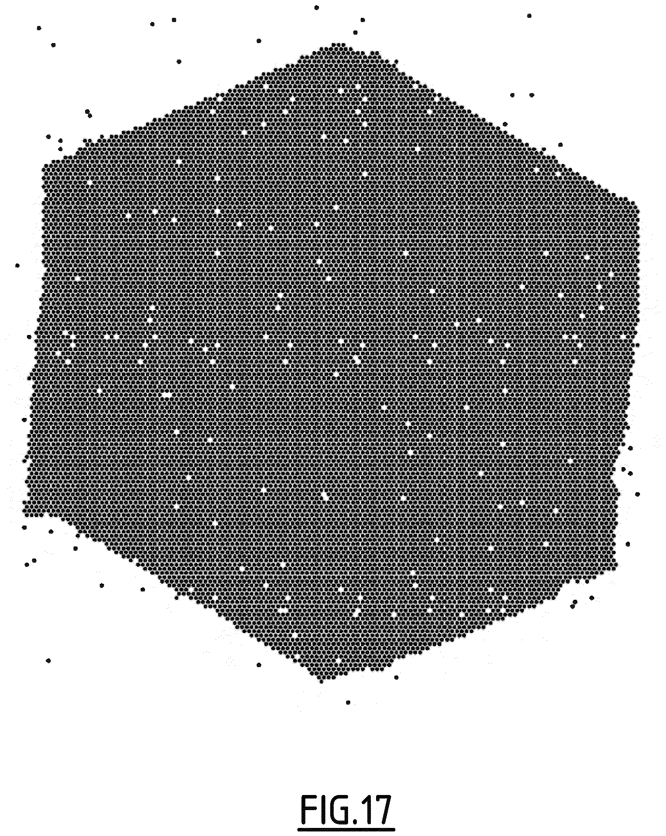

Figure 17 represents the spatial repartition in the microfluidic device of the 14,752 barcodes extracted from reads presenting a valid gene in the sequencing data of example 8. Each black circle represents a DNA spot from the DNA array whose barcode is associated with a least one cDNA in the sequencing data. Missing circles are DNA spots from the DNA array whose barcode was not found associated with a least one cDNA in the sequencing data. The hexagonal shape represented by the position of the recovered barcodes correspond to the area that was protected from the oxygen plasma during the microfluidic device fabrication. The majority of the spots in the protected area have been identified in the sequencing data.

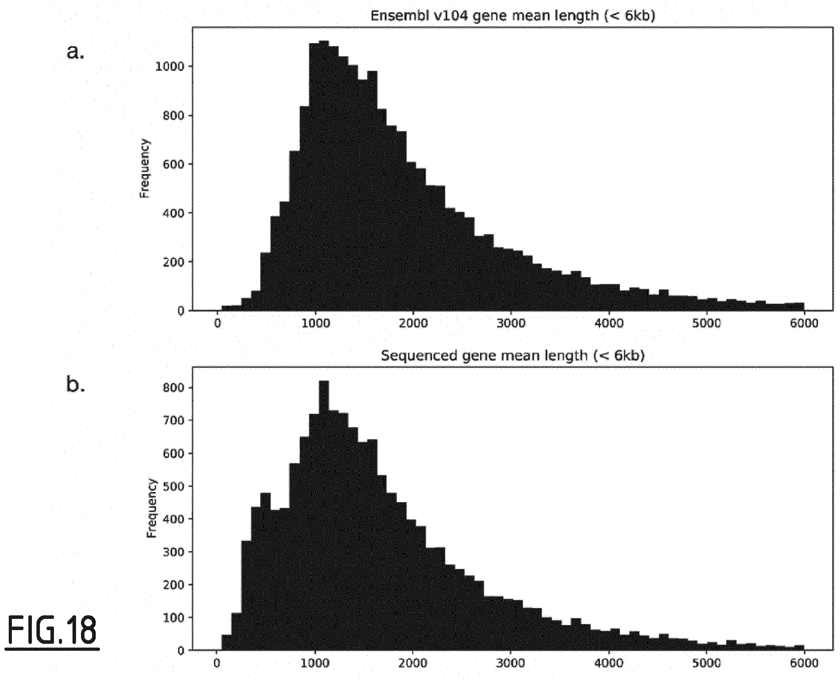

Figure 18 represents the length distribution of genes identified by sequencing in Example 8. Figure 18a represents the length distribution of the 20,465 genes annotated as protein coding gene in the assembly GRCh38.p13 Genocode 38 of the Genome Reference Consortium annotated by Ensembl version 104.38 (ref: http://may2021.archive.ensembl.org/Homo_sapiens/Info/Annotation). The gene length is determined as the sum of exons length. For each gene the means size of alternative spliced variants was used. Figure 18b represents the length distribution from the same database of only the 14,507 unique genes identified from reads presenting a valid gene and a valid barcode in the sequencing data of example 8. Similarity of the size distributions in panels a and b demonstrates an absence of size bias in gene recovery in example 8.

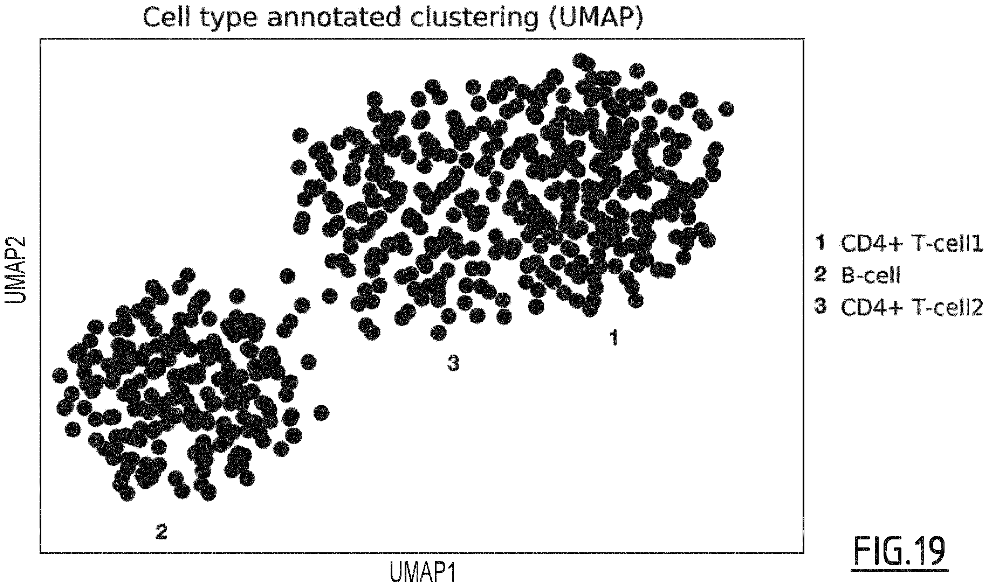

Figure 19 represents an annotated UMAP (McInnes, L, Healy, J, UMAP: Uniform Manifold Approximation and Projection for Dimension Reduction, ArXiv e-prints 1802.03426, 2018) for clustering of three repeats of example 8 with two different cell lines. RNA-seq described in example 8 was repeated twice with Jurkat human T-cells and once with Ramos human B-cells. For each experiment the 15,000 barcodes are randomly grouped to form 200 super barcodes in order to counter the shallow sequencing depth and the low number of cDNAs identified per barcode that would prevent the clustering. Then, Scanpy Python software (https://scanpy.readthedocs.io) is used to run a principal component analysis (PCA), then to detect clusters using the Leiden graph-clustering method (Traag et al., 2018, From Louvain to Leiden: guaranteeing well-connected communities) and to represent the clustering in two dimensions via UMAP graph before adding the annotation. The complete procedure is detailed on Scanpy website in the following link: https://scanpy-tutorials.readthedocs.io/en/latest/pbmc3k.html#Clustering-the-neighborhood-graph.

Two clusters are identified, one (cluster 1) made up of data from the two experiments with Jurkat cells (T cells) and the other (cluster 2) made up of data from the experiment with Ramos cells (B cells) demonstrating that the method can be used to identify different (closely related) cell types

### EXAMPLES

Oligonucleotides used in the examples have the sequences disclosed in Table 1.

**Table 1: Oligonucleotide structure**

| **Code** | **Name** | **Sequence (5' to 3')** | **Ab. Max** | **Em. Max** |
|---|---|---|---|---|
| A | Bmtl_U4_56FA M | /56FAM/TGCTAGCTCGCCACCGCAGC CACAA (SEQ ID NO:1) | 495nm | 520nm |
| B | UCP1w_PolydT 30_5Phos | /5Phos/CCACTCTAGCCGTTCGGAACN NNNNNNNNNNNTTTTTTTTTTTTTTT TTTTTTTTTTTTTTTVN (SEQ ID NO:2) | | |
| C | cg_fa_5TYE563 | /3TYE563/ACATGCGTTCTCTGG (SEQ ID NO:3) | 549nm | 563nm |
| D | sp_fb_5TYE665 | /5TYE665/TGGAAGGTTGAGCGA (SEQ ID NO:4) | 645nm | 665nm |
| E | sp_fc_5TYE705 | /5TYE705/CCGAACAACCAGACT (SEQ ID NO:5) | 686nm | 704nm |
| F | P7rGrG+G | CAAGCAGAAGACGGCATACGAGATrG rG+G (SEQ ID NO:6) | | |
| G | P5Rd1_U4c | AATGATACGGCGACCACCGAGATCTA CACTCTTTCCCTACACGACGCTCTTC CGATCTNNNNNNCTAGCGCGCAGTG AC (SEQ ID NO:7) | | |
| H | P7 | CAAGCAGAAGACGGCATACGAGAT (SEQ ID NO:8) | | |
| I | Bmtl_RD1c_36F AM | GTGTAGGGAAAGAGTGTGCTAGCGC ACAAT/36FAM/ (SEQ ID NO:9) | | |
| J | P5Rd1 | AATGATACGGCGACCACCGAGATCTA CACTCTTTCCCTACACGACGCTCTTC CGATCT (SEQ ID NO:10) | | |
| K | UCP1w_ASP4_ 5Phos | /5Phos/CCACTCTAGCCGTTCGGAACN NNNNNNNNNNNGCAATTTGTGAGGG CCGTTC (SEQ ID NO:11) | | |
| L | P7U1 | CAAGCAGAAGACGGCATACGAGATC CTTACCACTCGGCCATGCCGCCAA (SEQ ID NO:12) | | |
| M | ligA'_B' | CTCACGCGCAGACACGCGTTGCGCT G (SEQ ID NO:13) | | |
| N | ligB_UMI_Polyd T30 | GTCTGCGCGTGAGNNNNNNNNNNNN TTTTTTTTTTTTTTTTTTTTTTTTTTTTTT VN (SEQ ID NO:14) | | |
| O | GB_P5Rd1_Fs w | AATGATACGGCGACCACCGAGATCTA CACTCTTTCCCTACACGACGCTCTTC CGATCTNNNNNNCTAGCGCGCAGTG AC (SEQ ID NO:15) | | |
| P | P7_U4c | CAAGCAGAAGACGGCATACGAGATTT GTGGCTGCGGTGGCGAGCTAG (SEQ ID NO:16) | | |
| Q | Pre_Bcll_P5 | CATGCTTGATCAGACCACCGAGATCT AC (SEQ ID NO:17) | | |
| R | Phos_Rd1_Nxt_ UMI12_PolydT3 0VN | /5Phos/TCGTCGGCAGCGTCAGATGTG TATAAGAGACAGNNNNNNNNNNNNTT TTTTTTTTTTTTTTTTTTTTTTTTTTTTV N (SEQ ID NO:18) | | |
| S | Fluo_4.2_3Cy5 Sp | CATACTCGGTCTGCG/3Cy5Sp/ (SEQ ID NO:19) | 648nm | 668nm |
| T | Rd2_Nxt_9N | GTCTCGTGGGCTCGGAGATGTGTATA AGAGACAG NNNNNNNNN (SEQ ID NO:20) | | |
| U | P5 | AATGATACGGCGACCACCGAGATCTA CAC (SEQ ID NO:21) | | |
| V | P7_i05_Rd2_Nx t | CAAGCAGAAGACGGCATACGAGAT CCAGGAAG GTCTCGTGGGCTCGGAGATGTGTATA AGAGACAG (SEQ ID NO:22) | | |

### Example 1 - Total scRNA-seq in LCST cages with a DNA array

### Synthesis of the thermo-actuatable hydrogel

An ene-functionalised poly(n-isopropyle acrylamide) is synthesized following the steps descripted in the supplementary information of D'Eramo L.; et al., Microsystems & Nanoengineering, 2018, 4, 17069, doi:10.1038.

The swelling properties of the obtained polymer, as a function of temperature, are evaluated in various aqueous solutions: pure water, phosphate saline buffer, at pH2 and at pH 9. The results are given in Figure 7.

### Preparation of the first substrate

The first substrate, made of polydimethylsiloxane (PDMS), is prepared by standard soft lithography techniques, containing microstructures and a chamber. The height of the structures and the chamber depends on the objective and can be a couple of a tenth of a micron up to 100 microns tall.

### Functionalization of the first substrate

The PDMS substrate is exposed to Oxygen plasma for 50s after being cleaned with isopropanol. Immediately following the surface activation, a solution of anhydrous toluene with a 3 vol% of mercaptopropyltrimethoxysilane (ABCR Gelest) is put in contact with the substrate for 3 h inside a reactor under nitrogen. Following the thiol-modification of the surface, the substrate is rinsed with toluene and finally dried with nitrogen flow.

### Photo-patterning of hydrogel films onto the first substrate

Preformed functionalized pNIPAM (being ene-reactive) is spin-coated on the thiol-modified and micro-structured PDMS substrate with dithiol cross-linkers. A microvolume of a couple of 100µL of a solution of butanol and methanol (V/V = 1/1) containing the functionalized pNIPAM at a concentration between 3 and 15 wt.% and dithioerythritol (purchased from Sigma Aldrich, CAS number 3483-12-3) cross-linkers at a concentration between 3 and 10 wt.% is deposited onto the substrate. The conditions of spin-coating are fixed at an angular velocity varying between 500 rpm and 3000 rpm for a spinning time of 30s. The spread films are dried by heat in a 90°C oven for 5 minutes. The resulting layer thickness varies from a few tenth of a micron to 15 microns.

Chromium masks presenting numerous micro-structured cages are aligned with the design of the chamber and placed under UV lamp for deep UV exposure (8 watts, 250 nm wavelength). After exposure, free polymer chains are rinsed off by washing the substrate in an ultrapure water bath for 5 minutes. The hydrogel-patterned substrates are dried with nitrogen flow.

### Preparation of the second substrate

The second substrate used is a glass slide spotted with DNA strands (purchased from Agilent, referenced as an Agilent Microarray Format).

Presenting up to 1 million unique spots with different DNA strands grafted on it, this item provides different barcode on each spot. Each spot containing millions of DNA strands.

The micro array structure of the DNA is represented on Figure 8, each spot having a different barcode.

A localization system is integrated in the design of the array. Among the numerous unique spots, some of them carry a specific sequence for fluorescent labels capture (2 or more). They are placed in a way they form multiple shapes comprising triangle, square and circle as shown in Figure 9.

### Closure of the device

Bonding between the first and the second substrates is achieved by using an O2 plasma treatment for 50 s. A protective layer is tapped onto the area of interest avoiding Oxygen plasma to be active there. After the termination of the exposure, the PDMS substrate is placed on top of the DNA array so that the region of interest faces the hydrogel structures. A curing step is applied, for 30 minutes at least, by storing the chip inside a 70°C oven.

### Preparation of the chip for specific capture: total scRNA-seq

A solution of 100 mM Potassium Acetate; 30 mM HEPES, pH 7.5 and 20 µM of oligonucleotide A, B and C, D, E is injected inside the microfluidic chamber at 40°C (cages open) in order to hybridize the additional sequence of capture onto the seated DNA strands and to proceed to the localization step. The flow is stopped and the chip with the mixed oligonucleotides heated above 60°C for 2 minutes, then cooled down for 10 min at room temperature and rinsed with PBS solution at 37°C.

Fluorescent imaging at consecutive wavelength of 520nm, 563nm, 665nm, 704nm of the main chamber is performed, positioning fluorescent shapes and spots relative to the cages.

### Capture of the single cells

Tests were performed with different microfluidic geometries, all showing a common feature being a large chamber hosting the hydrogel cages.

A suspension of cells at a concentration of 10 million per ml with 1% Pluronic f68, 15% Optiprep and 1% BSA in TBS is prepared. Cells are injected inside the chip at 100µl/h; the chip being heated up at 37°C in order to open the cages. Once the cells are circulating around and above the cages, the flow is stopped, and the temperature is decreased to 20°C to close the cages. Cells are trapped inside the cages.

To perform the lysis, a solution of low salted water is injected inside the chamber through additional inlets that are not obstructed by the swollen hydrogel cages.

Cells' RNA strands are subsequently hybridized to the grafted sequence of capture as the aqueous phase around the cage is changed with PBS.

After a 10 minutes stop flow, a cleaning step is performed by opening the cage (increase temperature to 37°C) and flowing PBS inside the chamber.

After a thorough cleaning, a mix including 10U/µl of Reverse Transcriptase (Thermo Scientific Maxima H+), 0.5 mM dNTPs and 1U/µl of RiboLock RNase Inhibitor and 26µM of oligonucleotide F in RT buffer 1x is injected inside the chamber, followed by an immobilization of the flow to proceed to a retro-transcription of the captured strands for 2h at 55°C. The enzyme is immediately flushed with a PBS water solution to stop the RT.

A second mix containing enzyme Bmtl and Hi-T4 ligase in CutSmart buffer 1x supplemented with 1mM ATP is injected at 37°C and then stopped inside the chamber to ligate barcoded strands to the cDNA and to separate the cDNAs from the DNA array. The inner volume is then collected by pushing it out with a solution of PBS.

The collection tube is then incubated at 65°C for 20min to inactivate previously injected enzymes.

After an Exol treatment, a PCR amplification is performed on the collected sample using primers G and H.

The PCR product is then purified and quantified before being sequenced.

The demultiplexing of the sequencing data is performed looking at the barcode from position 30 to 44, at the UMI from position 65 to 76 and RNA transcript starting from position 109 of Read1.

### Example 2 - Targeted scRNA-seq in LCST cages with a DNA array

The microfluidic device is identical to example 1.

### Preparation of the chip for specific capture

Preparation is detailed in example 1, Poly(dT)VN of the 3' end of oligonucleotides B is replaced with gene specific sequences. A mix of oligonucleotides targeting different genes or different part of a gene can be used.

### Capture, lysis and library preparation

Capture, lysis and hybridization steps are detailed in example 1.

During the reverse transcription the 3' end rGrG+G of oligonucleotide F is replaced with gene specific sequences targeting similar gene as oligonucleotides B but in the reverse way. A mix of oligonucleotides targeting different genes or different part of a gene can be used

Steps from release to sequencing are detailed in example 1.

The demultiplexing of the sequencing data is performed as in example 1.

### Example 3 - Total scRNA-seq in UCST cages with a DNA array

### Synthesis of the thermos-actuatable hydrogel showing UCST behavior

A ene-functionalized UCST polymer is synthesized by free radical polymerization of methacrylamide (MA), acrylamide (AM) and allylmethacrylate (AMA) with 90:5:5 molar ratio using 2,2'-azobis(2-methylpropionamidine)dihydrochloride (V50) as thermal radical initiator. MA (8 g, 94 mmol), AM (0.371 g, 5.2 mmol), AMA (0.659 g, 5.2 mmol) and V50 (0.071 g, 0.3 mmol) were mixed in 247 mL of water and 123 mL of formamide. The solution was deoxygenated by nitrogen bubbling under the reflux condition at 55°C for 1 h. The medium was allowed to proceed under refluxed condition at 55 °C under nitrogen for 24 h. The polymer solution was then dialyzed in pure water for four days at 70°C. The ene-functionalized UCST P(MA-AM-AMA) terpolymer was finally recovered by freeze-drying.

The swelling properties of the obtained polymer, as a function of temperature, are evaluated in a phosphate saline buffer. The results are given in Figure 10.

### Preparation of the first substrate

Details are in example 1.

### Functionalization of the first substrate

Details are in example 1.

### Photo-patterning of hydrogel films onto the first substrate

P(MA-AM-AMA) terpolymer (ene-reactive UCST polymer) is spin-coated on the thiol-modified & micro-structured PDMS substrate with dithiol cross-linkers at a temperature of 40°C at least. A microvolume of a couple of 100uL of a solution of acetic acid (V/V = 1/1) containing P(MA-AM-AMA) polymer at a concentration between 3 and 15 %wt and dithioerythritol (purchased from Sigma Aldrich, CAS number 3483-12-3) cross-linkers at a concentration between 3 and 10 %wt is deposited onto the substrate. The conditions of spin-coating are fixed at an angular velocity varying between 500 rpm and 3000 rpm for a spinning time of 30s. The spread films are dried by heat in a 90°C oven for 5 minutes, in a water saturated environment. The resulting layer thickness varies from a few tenth of a micron to 15 microns.

Chromium masks presenting numerous microstructured cages are aligned with the design of the chamber and placed under UV lamp for deep UV exposure (8 watts, 250nm wavelength). After exposure, free polymer chains are rinsed off by washing the substrate in an ultrapure water bath for 5 minutes. The hydrogel-patterned substrates are dried with nitrogen flow.

### Preparation of the second substrate

Details are given in example 1.

### Preparation of the chip for specific capture: scRNA-seq

The preparation of the chip is detailed in example 1. Only temperatures of these steps differ: injection of the oligonucleotides and rinsing are performed at 20°C.

### Capture of the single cells

Capture step is detailed in example 1, with cells injection performed at 20°C. The cages are closed by heating the chip higher than 37°C.

Lysis and hybridization steps are detailed in example 1.

The cleaning step is performed in this case at room temperature.

Last steps are detailed in example 1.

### Example 4 - Total scRNA-seq with a film of LCST and a PDMS substrate hosting spotted strands in microwells

### Synthesis of the thermoactuatable hydrogel

Details in example 1.

### Preparation of the first substrate

A clean and new glass slide (1mm thick) is used as a first substrate.

### Functionalization of the first substrate

The thiol-modification of the substrate is done following the same protocol given in example 1.

### Photo-patterning of hydrogel films

The protocol is the same as those detailed in example 1 at the exception of the mask design, which is presenting a different design: a large rectangle matching the width and length of the microfluidic chamber.

### Preparation of the second substrate

The second substrate used is made of PDMS and is structured following standard techniques of softlithography. Inside the chamber has been structured an array of microwells, with a depth varying from few microns to 100 microns.

Based on Detection of harmful algal bloom causing microalgae using covalently immobilized capture oligonucleotide probes on glass and poly(dimethylsiloxane) surfaces (Analytical and Bioanalytical Chemistry. 2013. 10.1117/12.2034011), the substrate is functionalized in order to immobilize amine modified DNA strands inside the microwells.

Using standard DNA spotting protocols, 5' amine modified single DNA strands are deposited and thereafter grafted inside the microwells, having a different barcode in each microwell.

The structure of DNA microwells is represented on Figure 11.

A localization system is integrated in the design of the array, following the same protocol than in example 1.

### Closure of the device

The closure of the device is identical to example 1, knowing that the microarray to protect is on the PDMS substrate.

### Preparation of the chip for specific capture: scRNA-seq

A solution of 100 mM Potassium Acetate; 30 mM HEPES, pH 7.5 and 100 µM of oligonucleotide C, D, E and I is injected inside the microfluidic chamber at 40°C (cages open). The flow is stopped and the chip with the mixed oligonucleotides heated above 60°C for 2 minutes, then cooled down for 10 min at room temperature and rinsed with PBS solution at 40°C.

Fluorescent imaging for localization is performed like in example 1.

### Capture of the single cells

Capture, lysis, hybridization and RT steps are detailed in example 1.

A solution containing enzyme Bmtl in CutSmart buffer 1× is injected at 37°C and then stopped inside the chamber to ligate barcoded strands to the cDNA and to separate the cDNAs from the surface of the chip. The inner volume is then collected by pushing it out with a solution of PBS.

The collection tube is then incubated at 65°C for 20min to inactivate previously injected enzymes.

After an Exol treatment, a PCR amplification is performed on the collected sample using primers J and H.

The PCR product is then purified and quantified before being sequenced.

The demultiplexing of the sequencing data is performed looking at the barcode from position 1 to 15, at the UMI from position 16 to 27 and RNA transcript starting from position 60 of Read1.

### Example 5 - scCITE-seq in LCST cages with a DNA array

The microfluidic device is identical to example 1.

### Preparation of the chip for specific capture

Preparation is detailed in example 1, oligonucleotides B are supplemented with 10% of oligonucleotide K.

### Cell labeling

Cells are labeled with sub-population specific antibody carrying antibody specific barcode as showed in Figure 12.

The antibody is coupled to a streptavidin (e.g. using Streptavidin Conjugation Kit - Lightning-Link from abcam - ab102921). Prior to cell labeling, a biotin conjugated oligo, as described in figure 6 b., is added to the antibody solution in a 12:1 relation.

### Capture, lysis and library preparation

Capture, lysis and hybridization steps are detailed in example 1.

After a thorough cleaning, a mix including 10U/µl of Reverse Transcriptase (Thermo Scientific Maxima H+), 0.5 mM dNTPs and 1U/µl of RiboLock RNase Inhibitor and 26µM of oligonucleotide F in RT buffer 1× is injected inside the chamber, followed by an immobilization of the flow to proceed to a retro-transcription of the captured strands for 2h at 55°C. The enzyme is immediately flushed with a PBS solution to stop the RT.

A second mix containing enzyme Bmtl and Hi-T4 ligase in CutSmart buffer 1× supplemented with 1mM ATP is injected at 37°C and then stopped inside the chamber to ligate barcoded strands to the cDNA and to separate the cDNAs from the DNA array. The inner volume is then collected by flowing a solution of PBS.

The collection tube is then incubated at 65°C for 20min to inactivate previously injected enzymes.

After an Exol treatment, a PCR amplification is performed on the collected sample using primers G, H and L.

The PCR product is then purified and quantified before being sequenced.

The demultiplexing of the sequencing data is performed as in example 1, looking in addition at antibody tag starting from position 97 of Read1.

### Example 6 - Total scRNA-seq in LCST cages with a bridge type DNA array

### Preparation of the first substrate

The protocol is detailed in example 1.

### Preparation of the second substrate

The DNA array is detailed in example 1, harboring instead in this case the sequences represented on Figure 13.

### Closure of the device

Detailed in example 1.

### Preparation of the chip for specific capture: scRNA-seq

A solution containing the enzyme Nb.Bstl in CutSmart buffer 1× is injected inside the microfluidic chamber at 37°C (cages open) to proceed to the flipping step.

The flow is stopped for 10 min at 37°C and rinsed with PBS solution at 37°C.

A solution containing the enzyme Hi-T4 ligase in CutSmart buffer 1× supplemented with 1mM ATP and 20 µM of oligonucleotides M, N is injected inside the microfluidic chamber at 37°C (cages open) to proceed to the extension step.

The flow is stopped for 10 min at 37°C and rinsed with PBS solution at 37°C.

Then, a solution of 100 mM Potassium Acetate; 30 mM HEPES, pH 7.5 and 20 µM of oligonucleotide C, D, E is injected inside the microfluidic chamber at 40°C in order to hybridize the fluorescent sequences onto the seated DNA strands (localization step).

The flow is stopped and the chip with the mixed oligonucleotides heated above 60°C for 2 minutes, then cooled down for 10 min at room temperature and rinsed with PBS solution at 37°C.

Fluorescent imaging for localization is performed like in example 1.

### Capture, lysis and library preparation

Capture, lysis and hybridization steps are detailed in example 1.

After a thorough cleaning, a mix including 10U/µl of Reverse Transcriptase (Thermo Scientific Maxima H+), 0.5 mM dNTPs and 1U/µl of RiboLock RNase Inhibitor and 26µM of oligonucleotide F in RT buffer 1× is injected inside the chamber, followed by an immobilization of the flow to proceed to a retro-transcription of the captured strands for 2h at 55°C. The enzyme is immediately flushed with a PBS solution to stop the RT.

A second mix containing enzyme Nhel in CutSmart buffer 1× is injected at 37°C and then stopped inside the chamber to ligate barcoded strands to the cDNA and to separate the cDNAs from the DNA array. The inner volume is then collected by pushing it out with a solution of PBS.

The collection tube is then incubated at 65°C for 20min to inactivate previously injected enzymes.

After an Exol treatment, a PCR amplification is performed on the collected sample using primers O and H.

The PCR product is then purified and quantified before being sequenced.

The demultiplexing of the sequencing data is performed looking at the barcode from position 22 to 36, UMI from position 63 to 74 and RNA transcript starting from position 107 of Read1.

### Example 7 - scATAC-seq in UCST cages

### Preparation of the first substrate

The protocol is detailed in example 3.

### Preparation of the second substrate

The DNA array is detailed in example 3, harboring in this case the sequences represented on Figure 14.

### Closure of the device

Detailed in example 3.

### Preparation of the chip for specific capture: seATAC-seq

A solution containing the enzyme T4 Polynucleotide Kinase (NEB M0201) in 1X T4 Polynucleotide Kinase Reaction Buffer is injected inside the microfluidic chamber at 20°C (cages open) and incubated for 30min at 37°C to proceed to 5' phosphorylation of the strands anchored on the DNA array.

The chamber is then rinsed with PBS solution at 20°C.

A solution of 100 mM Potassium Acetate; 30 mM HEPES, pH 7.5 and 20 µM of oligonucleotides C, D, E is injected inside the microfluidic chamber at 20°C in order to hybridize the additional fluorescent sequences onto the seated DNA strands (localization step).

The flow is stopped and the chip with the mixed oligonucleotides heated above 60°C for 2 minutes, then cooled down for 10 min at room temperature and rinsed with PBS solution at 20°C.

Fluorescent imaging for localization is performed like in example 1.

A solution containing the enzyme phi29 in 1× phi29 DNA Polymerase Reaction Buffer with 20µm of oligonucleotide A is injected inside the microfluidic chamber at 20°C (cages open) to create a double stranded DNA adaptor (barcoded MEDS).

After 10 min incubation at 37°C, the chamber is rinsed with PBS solution at 20°C.

### Capture, lysis and library preparation

Capture and lysis are detailed in example 3.

A mix containing enzyme Bmtl in CutSmart buffer 1x is injected at 37°C and then stopped inside the chamber and incubated for 10 min at 37°C to release the dsDNA from the DNA array.

A mix including Nextera Tn5 Transposase (TDE1) in 1× TD reaction buffer is then injected inside the chamber at 37°C, followed by an immobilization of the flow for 30 min at 37°C to proceed to transposition.

The inner volume is then collected by flowing a solution of water at 20°C.

After an Exol treatment, a PCR amplification is performed on the collected sample using primers G and P.

The PCR product is then purified and quantified before being sequenced.

The demultiplexing of the sequencing data is performed looking at the barcode from position 30 to 44, and DNA transcript starting from position 64 of Read1.

Reference: Buenrostro, J., Wu, B., Litzenburger, U. et al. Single-cell chromatin accessibility reveals principles of regulatory variation. Nature 523, 486-490 (2015)

### Example 8-Total RNA-seq in LCST cages with a DNA array straing with purified RNA

### Synthesis of the thermo-actuatable hydrogel

Details are in example 1.

### Preparation of the first substrate

Details are in example 1.

### Functionalization of the first substrate

Details are in example 1.

### Photo-patterning of hydrogel films onto the first substrate

Details are in example 1.

### Preparation of the second substrate

The second substrate used is a glass slide spotted with DNA strands (purchased from Agilent, referenced as an Agilent Microarray Format).

Presenting up to 1 million unique spots with different DNA strands grafted on it, this item provides different barcode on each spot. Each spot containing millions of DNA strands.

The micro array structure of the DNA is represented on Figure 15, each spot having a different barcode.

A localization system is integrated in the design of the array. Among the numerous unique spots, some of them carry a specific sequence for capture of fluorescently-labeled DNA oligonucleotides by hybridization (2 or more). They are placed so as to form multiple shapes comprising triangle, square and circle as shown in Figure 9.

### Closure of the device

Details are in example 1.

### Preparation of the chip for specific capture: total RNA-seq

Oligonucleotides were design in silico and purchased from IDT, at 25 to 100 µM concentration in IDTE Buffer, pH 8.0, with standard desalting.

A solution of 100 mM Potassium Acetate, 30 mM HEPES, pH 7.5 and 10 µM of oligonucleotides Q, R and S is injected inside the microfluidic chamber at 40°C (cages open) in order to hybridize the additional sequence of capture onto the seated DNA strands and to proceed to the localization step. The flow is stopped and the chip with the mixed oligonucleotides heated above 60°C for 2 minutes, then incubated for 10 minutes at room temperature and rinsed with 1× SSC solution (Thermo Scientific #15413549) at 40°C.

A brightfield and a fluorescent imaging (excitation at 650 nm and emission at 670 nm) of the main chamber is performed. An informatic identification of the DNA array spots and cage is performed as shown in figure 16. The identification is based on the superposition of both images. The brightfield image allows to identify the cages. The fluorescent image, represented by the black circles A, B and C in figure 16, makes it possible to identify certain spots on which fluorescent DNA probes have been previously hybridized. The relative position of these circles define a unique location on the chip, allowing to map the original organization of the DNA array onto the superposed images. Finally, thanks to this identification, we can associate spot specific barcodes with each cage.

### Primer synthesis.

A mix containing Sulfolobus DNA Polymerase IV (NEB #M0327S) and Hi-T4 ligase (NEB #M2622S) in Thermopol 1x (NEB #B9004S) supplemented with 1 mM ATP (NEB #P0756S) is injected at 40°C and then incubated at 50°C for 2 hours inside the chamber to polymerize and ligate barcoded primers. The inner volume is then rinsed with 1x SSC solution at 40°C (Thermo Scientific #15413549).

### Cell culture

Jurkat human T lymphocyte ATCC^{®} TIB-152 are cultivated in RPMI 1640 Medium (Gibco #61870044) supplemented with 10% heat inactivated Fetal Bovine Serum (Gibco #10082147) and 1% Penicillin-Streptomycin (Gibco #15140122). Cells are seeded on 25 cm² or 75 cm² culture flask at 37°C with 5% CO₂ following ATCC recommendations. On reaching 75-80% confluence the cells are diluted. After retrieving from cell culture, the cells are finally centrifuged at 130 r.c.f .for 5 minutes.

### Capture of the mRNA strands

Total RNA was purified from Jurkat cells using Qiagen RNeasy Mini Kit (Qiagen #74104) and injected at 40°C with 1x SSC and 1U/µl of RNAse inhibitor (Thermo Scientific #11581505). The microfluidic device is then incubated 5 minutes at 65°C, then ramped down to 4°C followed by 5 minutes at 25°C. mRNA strands are subsequently hybridized to the grafted sequence of capture.

### Barcoded cDNA synthesis

A cleaning step is performed by opening the cage at 37°C and flowing a volume of RT buffer (Thermo Scientific #EP0742) equivalent of 20 times the inner volume of the chamber through the chamber for 3 minutes.

After cleaning, a mix including 10U/µl of Reverse Transcriptase (Thermo Scientific #EP0742), 0.5 mM dNTPs (NEB #N0447S) and 1U/µl of RNase Inhibitor (Thermo Scientific #11581505) in 1× RT buffer is injected inside the chamber, followed by an immobilization of the flow and reverse transcription of the captured strands for 2h at 50°C. The enzyme is immediately flushed with a 1× SSC solution to stop reverse transcription.

A second mix containing 2U/µl of exonuclease I in 1× Exonuclease I Reaction Buffer (NEB #M0293S) is injected and incubated at 37°C for 30 minutes. The enzyme is immediately flushed with a 1× SSC solution to stop the reaction.

DNase/RNase-Free Distilled Water (Invitrogen #10977023) is injected in the chamber and heated up to 98°C to dehybridize synthesized cDNA. The inner volume is then collected by flowing additional water.

10 µL of NEB2 Buffer (NEB #M0212L) and 10 µL of 10 µM of oligonucleotide T is added to 65 uL of the recovered sample.

The solution is incubated at 95°C for 2 minutes and transferred immediately to ice. Then, 8 µL of 10 nM dNTPs and 7 µL of Klenow exo- (NEB #M0212L) is added.

The mix is placed in a thermocycler pre-chilled at 4°C, the temperature ramped slowly to 37°C, and hold for 30 minutes to perform the synthesis of the second strand.

cDNAs are then purified using SPRIselect magnetic beads (Beckman #B23317) following the manufacturer's instructions.

A 2-step PCR amplification with an elongation of 30 seconds is then performed for 15 cycles on the purified sample using primers G and H and Q5 High-Fidelity DNA Polymerase (NEB #M0491).

The PCR product is purified using SPRIselect magnetic beads (Beckman #B23317) and quantified before being sequenced using an Illumina sequencing platform.

The demultiplexing of the sequencing data is performed by retrieving for each read the barcodes from position 1 to 15 of Index1 reads, the UMI from position 1 to 12 of Read1 reads and the RNA transcript starting from position 1 of Read2 reads.

Out of the 1,504,957 complete sequenced reads, we have identified 738,483 unique cDNAs determined as reads with a unique barcode, a unique gene and a unique UMI. We have identified a mean of 102 reads per barcode and a median of 50 UMI per barcode. The sequencing was not deep enough to be saturating, as shown by the low number of reads per UMI.

Figure 17 represents the spatial repartition in the microfluidic device of the 14,752 barcodes extracted from reads presenting a valid gene in the sequencing data. Each black circle represents a DNA spot from the DNA array whose barcode is associated with a least one cDNA in the sequencing data. Missing circles are DNA spots from the DNA array whose barcode was not found associated with a least one cDNA in the sequencing data. The hexagonal shape represented by the position of the recovered barcodes correspond to the area that was protected from the oxygen plasma during the microfluidic device fabrication. The majority of the spots in the protected area have been identified in the sequencing data.

14,507 unique genes were extracted from reads presenting a valid barcode. Figure 18 represents the length distribution of genes identified by sequencing in Example 8. Panel a represents the length distribution of the 20,465 genes annotated as protein coding gene in the assembly GRCh38.p13 Genocode 38 of the Genome Reference Consortium annotated by Ensembl version 104.38 (ref: http://may2021.archive.ensembl.org/Homo_sapiens/Info/Annotation). The gene length is determined as the sum of exons length. For each gene the means size of alternative spliced variants was used. Panel b represents the length distribution from the same database of only the 14,507 unique genes identified from reads presenting a valid gene and a valid barcode in the sequencing data. Similarity of the size distrubitions in panels a and b demonstrates an absence of size bias in gene recovery.

The was repeated twice with Jurkat cells and once with Ramos human B lymphocyte ATCC^{®} CRL-1923 cells, cultivated in similar conditions as Jurkat cells. Figure 19 represents an annotated UMAP (McInnes, L, Healy, J, UMAP: Uniform Manifold Approximation and Projection for Dimension Reduction, ArXiv e-prints 1802.03426, 2018) for clustering of three repeats of example 8 with two different cell lines. RNA-seq described in example 8 was repeated twice with Jurkat human T-cells and once with Ramos human B-cells. For each experiment the 15,000 barcodes are randomly grouped to form 200 super barcodes in order to counter the shallow sequencing depth and the low number of cDNAs identified per barcode that would prevent the clustering. Then, Scanpy Python software (https://scanpy.readthedocs.io) is used to run a principal component analysis (PCA), then to detect clusters using the Leiden graph-clustering method (Traag et al., 2018, From Louvain to Leiden: guaranteeing well-connected communities) and to represent the clustering in two dimensions via UMAP graph before adding the annotation. The complete procedure is detailed on Scanpy website in the following link: https://scanpy-tutorials.readthedocs.io/en/latest/pbmc3k.html#Clustering-the-neighborhood-graph.

Two clusters are identified, one (cluster 1) made up of data from the two experiments with Jurkat cells (T cells) and the other (cluster 2) made up of data from the experiment with Ramos cells (B cells) demonstrating that the method can be used to identify different (closely related) cell types.

### Example 9 - Total scRNA-seq in LCST cages with a DNA array starting with a mix of Jurkat and Ramos human cells.

The procedure is identical to example 8 except that instead of injecting purified RNA in the microfluidic device, the cells are injected, capture and lysed in the chip as described in example 1.

## Claims

1. A microfluidic device (10) comprising:
- a first wall (12) comprising a first substrate (14) on which a plurality of closed patterns (16) is grafted,
- a second wall (18), facing the first wall (12), comprising a second substrate (20),
- a plurality of nucleic acids (22) grafted either on the first substrate (14) or on the second substrate (20), wherein each nucleic acid (22) comprises a barcode that encodes the position of the nucleic acid on said first (14) or second (20) substrate,
wherein at least the plurality of closed patterns (16) or the second substrate (20) is made of an actuatable hydrogel which is swellable between a retracted state and a swollen state in which the closed patterns (16) and the second substrate (20) come into contact.

2. The microfluidic device (10) according to claim 1, wherein the actuatable swellable hydrogel is a temperature-responsive swellable hydrogel.

3. The microfluidic device (10) according to claim 2, wherein the temperature-responsive hydrogel has a critical solution temperature ranging from 4°C to 98°C, preferably from 20°C to 50°C, more preferably from 25°C to 40°C.

4. The microfluidic device (10) according to claim 3, wherein the critical solution temperature is a lower critical solution temperature above which the temperature-responsive hydrogel is in the retracted state and below which the temperature-responsive hydrogel is in the swollen state.

5. The microfluidic device (10) according to claim 3, wherein the critical solution temperature is an upper critical solution temperature above which the temperature-responsive hydrogel is in the swollen state and below which the temperature-responsive hydrogel is in the retracted state.

6. The microfluidic device (10) according to any of claims 1 to 5, wherein the polymer matrix of the hydrogel comprises, preferably consists of, a thermo-responsive polymer chosen from homopolymers, copolymers and terpolymers of acrylic acid, alkyl (meth)acrylates, alkyl (meth)acrylamides, oligoethylene (meth)acrylates, sulfobetaines (meth)acrylates and N-acryloyl glycinamide and any mixtures thereof, preferably chosen from homopolymers copolymers and terpolymers of alkyl (meth)acrylamides and any mixtures thereof, more preferably the polymer is poly(N-lsopropylacrylamide).

7. The microfluidic device (10) according to any of claims 1 to 6, further comprising at least one inlet (24) and at least one outlet (26) permitting respectively the introduction or the removal of reactants into the device (10).

8. The microfluidic device (10) according to any of claims 1 to 7, wherein the first substrate (14) and the second substrate (20) are independently made in a material chosen from: silicon, quartz, glass, polydimethylsiloxane, thermoplastics such as cyclic olefin copolymers and polycarbonates, preferably from glass and polydimethylsiloxane.

9. The microfluidic device (10) according to any of claims 1 to 8, wherein the plurality of closed patterns (16) is made of the actuatable swellable hydrogel and the second substrate (20) is made of a non-swellable material.

10. The microfluidic device (10) according to any of claims 1 to 8, wherein the second substrate (20) is made of the actuatable swellable hydrogel and the closed patterns (16) are made of a non-swellable material.

11. The microfluidic device (10) according to any of claims 1 to 10, wherein a plurality of ligands is grafted on the first substrate (14) and/or on the second substrate (20).

12. The microfluidic device (10) according to any of claims 1 to 10, wherein a plurality of ligands conjugated with a nucleic acid is associated by hybridization to at least part of the grafted nucleic acids (22).

13. The microfluidic device (10) according to claim 11 or 12, wherein each ligand is independently chosen from the group consisting of antibodies, fragments of antibody, lectins, and aptamers.

14. The microfluidic device (10) according to any of claims 1 to 13, wherein nucleic acids (22) sharing the same barcode have a plurality of sequences.

15. The microfluidic device (10) according to any of claims 1 to 14, wherein nucleic acids (22) comprise one or any combinations of the following sequences:
1) a restriction site or a photocleavable site for nucleic acid release,
2) a sequence complementary to an amplification primer for further amplification,
3) a T7 RNA polymerase promoter sequence for in vitro transcription (IVT),
4) a hybridization site, a ligation site or a recombination site, for nucleic acid labeling, and
5) a sequence of randomized nucleotide residues that function as a unique molecular identifier (UMI).

16. A method of manufacture of a device (10) as defined in claims 1 to 15, said method comprising:
a) Providing a first substrate (14),
b) Grafting on the surface of said first substrate (14) a plurality of closed patterns (16),
c) Providing a second substrate (20),
d) Grafting a plurality of nucleic acids (22) either on the surface of the first substrate (14), or on the surface of the second substrate (20), wherein each nucleic acid (22) comprises a barcode that encodes the position of the nucleic acid (22) on said first (14) or second (20) substrate;
e) Positioning the first substrate (14) and the second substrate (20) by placing the closed patterns (16) and the nucleic acids (22) between the first substrate (14) and the second substrate (20),
f) Bonding the first (14) and the second (20) substrates.

17. The method according to claim 16, further comprising at least one of the following steps:
A) grafting a plurality of ligands on the surface of the first substrate (14) and/or on the surface of the second substrate (20), and/or
B) associating to the plurality of grafted nucleic acids (22) a plurality of ligands by hybridization, the plurality of ligands being conjugated with a nucleic acid being complementary to at least a part of the grafted nucleic acids (22), and/or
C) coating an adhesion coating on at least part of the surface of the first substrate (14) and/or of the second substrate (20), and associating to said adhesion coating a plurality of ligands by non-covalent binding.

18. The method according to any of claims 16 and 17, wherein said method further comprises one or more of the following steps:
1) Hybridizing a DNA comprising a sequence complementary to all or part of a constant sequence present in grafted nucleic acids (22);
2) Extending the hybridizing DNA by polymerization;
3) Ligating the grafted nucleic acids (22) with a DNA sequence;
4) Releasing all or part of the grafted nucleic acid (22), possibly previously modified by hybridization, extension or ligation according to 1), 2) or 3), from the surface of the first (14) or second (20) substrate, by cleavage.

19. A method of performing analysis of cells or organelles comprising:
a) Providing a microfluidic device (10) as defined in claims 1 to 15 and a preparation of cells or organelles;
b) Optionally, associating all or part of the cells or organelles with a common labeling nucleic-acid sequence or with a plurality of different labeling nucleic-acid sequences;
c) Injecting in the microfluidic device (10) the cells or organelles in suspension under conditions in which the hydrogel is in retracted state;
d) Modifying the conditions to actuate the hydrogel into swollen state, thereby trapping cells or organelles in a cage formed by the first (14) and second (20) walls of the microfluidic device (10), and the closed pattern (16) of hydrogel in swollen state;
e) Optionally analyzing captured cells or organelles and/or molecules they secrete, using optical imaging;
f) Optionally releasing in the cage, the grafted nucleic acids (22) from the surface of the first (14) or second (20) substrate of the microfluidic device (10);
g) Optionally, lysing trapped cells or organelles, thereby releasing cellular or organellar nucleic acids in the cages;
h) Associating the barcode of the nucleic acids (22) with either the released cellular or organellar nucleic acids and/or labeling nucleic-acid sequence(s) thereby forming barcoded nucleic acids;
i) Modifying the conditions to actuate the hydrogel into the retracted state;
j) Releasing the grafted nucleic acids (22) from the first (14) or second (20) substrate of the microfluidic device (10), if not released in f);
k) Recovering and sequencing the barcoded nucleic acids;
l) Optionally mapping the barcoded sequencing data onto the data from optical imaging obtained in e).

20. The method of performing analysis of cells or organelles according to claim 19, wherein said method further comprises:
e1) Binding of an analyte or of analytes secreted or released by the captured cells or organelles to ligands grafted directly or indirectly to the surface of the first substrate (14) and/or on the surface of the second substrate (20);
e2) Detecting the analyte or analytes bound to the grafted ligand by binding with a labeled second ligand or labeled ligands that is/are specific for the bound analyte or analytes.

21. The method of performing analysis of cells or organelles according to claim 20, wherein at step e2), detecting is performed
i) directly with a second ligand or ligands fluorescently labeled; or
ii) indirectly, with a second ligand or ligands labeled with a ligand identification nucleic acid that is specific to the analyte or analytes that is(are) bound to the grafted ligand, wherein the sequence of said ligand identification nucleic acid allows identification of the ligand and the analyte or analytes bound to the grafted ligand and becomes associated with the barcode of the nucleic acid (22), thereby forming barcoded nucleic acids.

## Patentansprüche

1. Mikrofluidvorrichtung (10), umfassend:
- eine erste Wand (12), die ein erstes Substrat (14) umfasst, auf dem eine Vielzahl geschlossener Muster (16) aufgepfropft ist,
- eine der ersten Wand (12) zugewandte zweite Wand (18), die ein zweites Substrat (20) umfasst,
- eine Vielzahl von Nukleinsäuren (22), die entweder auf dem ersten Substrat (14) oder auf dem zweiten Substrat (20) aufgepfropft sind, wobei jede Nukleinsäure (22) einen Barcode umfasst, der die Position der Nukleinsäure auf dem ersten (14) oder zweiten (20) Substrat codiert,
wobei mindestens die Vielzahl geschlossener Muster (16) oder das zweite Substrat (20) aus einem aktivierbaren Hydrogel hergestellt ist, das zwischen einem zurückgezogenen Zustand und einem gequollenen Zustand, in dem die geschlossenen Muster (16) und das zweite Substrat (20) in Kontakt kommen, quellbar ist.

2. Mikrofluidvorrichtung (10) nach Anspruch 1, wobei das aktivierbare quellbare Hydrogel ein temperaturempfindliches quellbares Hydrogel ist.

3. Mikrofluidvorrichtung (10) nach Anspruch 2, wobei das temperaturempfindliche Hydrogel eine kritische Lösungstemperatur im Bereich von 4 °C bis 98 °C, vorzugsweise von 20 °C bis 50 °C, mehr bevorzugt von 25 °C bis 40 °C aufweist.

4. Mikrofluidvorrichtung (10) nach Anspruch 3, wobei die kritische Lösungstemperatur eine untere kritische Lösungstemperatur ist, oberhalb derer das temperaturempfindliche Hydrogel in dem zurückgezogenen Zustand vorliegt und unterhalb derer das temperaturempfindliche Hydrogel in dem gequollenen Zustand vorliegt.

5. Mikrofluidvorrichtung (10) nach Anspruch 3, wobei die kritische Lösungstemperatur eine obere kritische Lösungstemperatur ist, oberhalb derer das temperaturempfindliche Hydrogel in dem gequollenen Zustand vorliegt und unterhalb derer das temperaturempfindliche Hydrogel in dem zurückgezogenen Zustand vorliegt.

6. Mikrofluidvorrichtung (10) nach einem der Ansprüche 1 bis 5, wobei die Polymermatrix des Hydrogels ein thermoempfindliches Polymer umfasst, vorzugsweise daraus besteht, das ausgewählt ist aus Homopolymeren, Copolymeren und Terpolymeren von Acrylsäure, Alkyl(meth)acrylaten, Alkyl(meth)acrylamiden, Oligoethylen(meth)acrylaten, Sulfobetain(meth)acrylaten und N-Acryloylglycinamid und beliebigen Mischungen davon, vorzugsweise ausgewählt aus Homopolymeren, Copolymeren und Terpolymeren von Alkyl(meth)acrylamiden und beliebigen Mischungen davon, wobei das Polymer mehr bevorzugt Poly(N-isopropylacrylamid) ist.

7. Mikrofluidvorrichtung (10) nach einem der Ansprüche 1 bis 6, ferner umfassend mindestens einen Einlass (24) und mindestens einen Auslass (26), die jeweils die Einführung oder die Entfernung von Reaktanten in die Vorrichtung (10) ermöglichen.

8. Mikrofluidvorrichtung (10) nach einem der Ansprüche 1 bis 7, wobei das erste Substrat (14) und das zweite Substrat (20) unabhängig aus einem Material hergestellt sind, ausgewählt aus: Silicium, Quarz, Glas, Polydimethylsiloxan, Thermoplasten wie cyclischen Olefin-Copolymeren und Polycarbonaten, vorzugsweise aus Glas und Polydimethylsiloxan.

9. Mikrofluidvorrichtung (10) nach einem der Ansprüche 1 bis 8, wobei die Vielzahl geschlossener Muster (16) aus dem aktivierbaren, quellbaren Hydrogel und das zweite Substrat (20) aus einem nicht quellbaren Material hergestellt ist.

10. Mikrofluidvorrichtung (10) nach einem der Ansprüche 1 bis 8, wobei das zweite Substrat (20) aus dem aktivierbaren, quellbaren Hydrogel hergestellt ist und die geschlossenen Muster (16) aus einem nicht quellbaren Material hergestellt sind.

11. Mikrofluidvorrichtung (10) nach einem der Ansprüche 1 bis 10, wobei eine Vielzahl von Liganden auf dem ersten Substrat (14) und/oder auf dem zweiten Substrat (20) aufgepfropft ist.

12. Mikrofluidvorrichtung (10) nach einem der Ansprüche 1 bis 10, wobei eine Vielzahl von mit einer Nukleinsäure konjugierten Liganden durch Hybridisierung mit zumindest einem Teil der aufgepfropften Nukleinsäuren (22) assoziiert ist.

13. Mikrofluidvorrichtung (10) nach Anspruch 11 oder 12, wobei jeder Ligand unabhängig aus der Gruppe ausgewählt ist, die aus Antikörpern, Antikörperfragmenten, Lektinen und Aptameren besteht.

14. Mikrofluidvorrichtung (10) nach einem der Ansprüche 1 bis 13, wobei Nukleinsäuren (22), die den gleichen Barcode teilen, eine Vielzahl von Sequenzen aufweisen.

15. Mikrofluidvorrichtung (10) nach einem der Ansprüche 1 bis 14, wobei die Nukleinsäuren (22) eine oder beliebige Kombinationen der folgenden Sequenzen umfassen:
1) eine Restriktionsstelle oder eine photospaltbare Stelle zur Freisetzung von Nukleinsäure,
2) eine zu einem Amplifikationsprimer komplementäre Sequenz zur weiteren Amplifikation,
3) eine T7-RNA-Polymerase-Promotorsequenz für die In-vitro-Transkription (IVT),
4) eine Hybridisierungsstelle, eine Ligationsstelle oder eine Rekombinationsstelle zur Nukleinsäuremarkierung und
5) eine Sequenz randomisierter Nukleotidreste, die als ein eindeutiger molekularer Identifikator (UMI) fungieren.

16. Verfahren zum Fertigen einer Vorrichtung (10) wie in den Ansprüchen 1 bis 15 definiert, wobei das Verfahren umfasst:
a) Bereitstellen eines ersten Substrats (14),
b) Aufpfropfen einer Vielzahl geschlossener Muster (16) auf die Oberfläche des ersten Substrats (14),
c) Bereitstellen eines zweiten Substrats (20),
d) Aufpfropfen einer Vielzahl von Nukleinsäuren (22) entweder auf die Oberfläche des ersten Substrats (14) oder auf die Oberfläche des zweiten Substrats (20), wobei jede Nukleinsäure (22) einen Barcode umfasst, der die Position der Nukleinsäure (22) auf dem ersten (14) oder zweiten (20) Substrat codiert,
e) Positionieren des ersten Substrats (14) und des zweiten Substrats (20) durch Platzieren der geschlossenen Muster (16) und der Nukleinsäuren (22) zwischen das erste Substrat (14) und das zweite Substrat (20),
f) Verbinden des ersten (14) und des zweiten (20) Substrats.

17. Verfahren nach Anspruch 16, ferner umfassend mindestens einen der folgenden Schritte:
A) Aufpfropfen einer Vielzahl von Liganden auf die Oberfläche des ersten Substrats (14) und/oder auf die Oberfläche des zweiten Substrats (20), und/oder
B) Assoziieren einer Vielzahl von Liganden mit der Vielzahl von aufgepfropften Nukleinsäuren (22) durch Hybridisierung, wobei die Vielzahl von Liganden mit einer Nukleinsäure konjugiert wird, die komplementär zu zumindest einem Teil der aufgepfropften Nukleinsäuren (22) ist, und/oder
C) Überziehen einer Haftbeschichtung auf zumindest einen Teil der Oberfläche des ersten Substrats (14) und/oder des zweiten Substrats (20) und Assoziieren einer Vielzahl von Liganden mit der Haftbeschichtung durch nicht kovalentes Binden.

18. Verfahren nach einem der Ansprüche 16 und 17, wobei das Verfahren ferner einen oder mehrere der folgenden Schritte umfasst:
1) Hybridisieren einer DNA, die eine Sequenz umfasst, die zu einer in den aufgepfropften Nukleinsäuren (22) vorhandenen konstanten Sequenz ganz oder zum Teil komplementär ist;
2) Verlängern der hybridisierenden DNA durch Polymerisation;
3) Ligieren der aufgepfropften Nukleinsäuren (22) mit einer DNA-Sequenz;
4) Freisetzen der ganzen oder eines Teils der aufgepfropften Nukleinsäure (22), die möglicherweise zuvor durch Hybridisieren, Verlängern oder Ligieren gemäß 1), 2) oder 3) modifiziert wurde, von der Oberfläche des ersten (14) oder zweiten (20) Substrats durch Spaltung.

19. Verfahren zum Durchführen einer Analyse von Zellen oder Organellen, umfassend:
a) Bereitstellen einer Mikrofluidvorrichtung (10) wie in den Ansprüchen 1 bis 15 definiert und einer Präparation von Zellen oder Organellen;
b) optional, Assoziieren der ganzen oder eines Teils der Zellen oder Organellen mit einer gemeinsamen Markierungs-Nukleinsäuresequenz oder mit einer Vielzahl unterschiedlicher Markierungs-Nukleinsäuresequenzen;
c) Injizieren der Zellen oder Organellen in Suspension in die Mikrofluidvorrichtung (10) unter Bedingungen, bei denen das Hydrogel im zurückgezogenen Zustand vorliegt;
d) Modifizieren der Bedingungen, um das Hydrogel in den gequollenen Zustand zu aktivieren, wodurch Zellen oder Organellen in einem Käfig gefangen werden, der durch die erste (14) und zweite (20) Wand der Mikrofluidvorrichtung (10) und das geschlossene Muster (16) des Hydrogels im gequollenen Zustand gebildet wird;
e) optional, Analysieren eingefangener Zellen oder Organellen und/oder von Molekülen, die sie absondern, unter Verwendung von optischer Bildgebung;
f) optional, Freisetzen der aufgepfropften Nukleinsäuren (22) in dem Käfig von der Oberfläche des ersten (14) oder zweiten (20) Substrats der Mikrofluidvorrichtung (10);
g) optional, Lysieren gefangener Zellen oder Organellen, wodurch zelluläre oder organelläre Nukleinsäuren in den Käfigen freigesetzt werden;
h) Assoziieren des Barcodes der Nukleinsäuren (22) entweder mit den freigesetzten zellulären oder organellären Nukleinsäuren und/oder Markierungs-Nukleinsäuresequenz(en), wodurch mit Barcodes versehene Nukleinsäuren gebildet werden;
i) Modifizieren der Bedingungen, um das Hydrogel in den zurückgezogenen Zustand zu aktivieren;
j) Freisetzen der aufgepfropften Nukleinsäuren (22) von dem ersten (14) oder zweiten (20) Substrat der Mikrofluidvorrichtung (10), sofern nicht in f) freigesetzt;
k) Rückgewinnen und Sequenzieren der mit Barcode versehenen Nukleinsäuren;
l) optional, Abbilden der mit Barcode versehenen Sequenzierungsdaten auf den in e) erhaltenen Daten aus der optischen Bildgebung.

20. Verfahren zum Durchführen einer Analyse von Zellen oder Organellen nach Anspruch 19, wobei das Verfahren ferner umfasst:
e1) Binden eines Analyten oder von Analyten, die von den eingefangenen Zellen oder Organellen abgesondert oder freigesetzt werden, an Liganden, die direkt oder indirekt auf die Oberfläche des ersten Substrats (14) und/oder auf die Oberfläche des zweiten Substrats (20) aufgepfropft sind;
e2) Nachweisen des Analyten oder der Analyten, der/die an den aufgepfropften Liganden gebunden ist/sind, durch Binden mit einem markierten zweiten Liganden oder mit markierten Liganden, der/die spezifisch für den gebundenen Analyten oder die gebundenen Analyten ist/sind.

21. Verfahren zum Durchführen einer Analyse von Zellen oder Organellen nach Anspruch 20, wobei bei Schritt e2) das Nachweisen folgendermaßen durchgeführt wird:
i) direkt mit einem zweiten Liganden oder mit Liganden, die fluoreszenzmarkiert sind; oder
ii) indirekt mit einem zweiten Liganden oder mit Liganden, die mit einer Ligandenidentifizierungsnukleinsäure markiert sind, die für den Analyten oder die Analyten spezifisch ist, der/die an den aufgepfropften Liganden gebunden ist/sind, wobei die Sequenz der Ligandenidentifizierungsnukleinsäure die Identifizierung des Liganden und des Analyten oder der Analyten, die an den aufgepfropften Liganden gebunden sind, erlaubt und mit dem Barcode der Nukleinsäure (22) assoziiert wird, wodurch mit Barcode versehene Nukleinsäuren gebildet werden.

## Revendications

1. Dispositif microfluidique (10) comprenant :
- une première paroi (12) comprenant un premier substrat (14) sur lequel une pluralité de motifs fermés (16) est greffée,
- une seconde paroi (18), faisant face vers la première paroi (12), comprenant un second substrat (20),
- une pluralité d'acides nucléiques (22) greffés soit sur le premier substrat (14) soit sur le second substrat (20), dans lequel chaque acide nucléique (22) comprend un code-barres qui encode la position de l'acide nucléique sur ledit premier (14) ou second (20) substrat,
dans lequel au moins la pluralité de motifs fermés (16) ou le second substrat (20) est fabriqué en hydrogel actionnable qui peut gonfler entre un état rétracté et un état gonflé dans lequel les motifs fermés (16) et le second substrat (20) viennent en contact.

2. Dispositif microfluidique (10) selon la revendication 1, dans lequel l'hydrogel gonflable actionnable est un hydrogel gonflable réagissant à la température.

3. Dispositif microfluidique (10) selon la revendication 2, dans lequel l'hydrogel gonflable réagissant à la température a une température de solution critique allant de 4 °C à 98 °C, de préférence de 20 °C à 50 °C, plus préférablement de 25 °C à 40 °C.

4. Dispositif microfluidique (10) selon la revendication 3, dans lequel la température de solution critique est une température de solution critique inférieure au-dessus de laquelle l'hydrogel réagissant à la température est dans l'état rétracté et en dessous de laquelle l'hydrogel réagissant à la température est dans l'état gonflé.

5. Dispositif microfluidique (10) selon la revendication 3, dans lequel la température de solution critique est une température de solution critique supérieure au-dessus de laquelle l'hydrogel réagissant à la température est dans l'état gonflé et en dessous de laquelle l'hydrogel réagissant à la température est dans l'état rétracté.

6. Dispositif microfluidique (10) selon l'une quelconque des revendications 1 à 5, dans lequel la matrice polymère de l'hydrogel comprend, de préférence est constituée de, un polymère thermoréactif choisi parmi des homopolymères, copolymères et terpolymères d'acide acrylique, (méth)acrylates d'alkyle, (méth)acrylamides d'alkyle, (méth)acrylates d'oligoéthylène, (méth)acrylates de sulfobétaines et N-acryloylglycinamide et de quelconques mélanges de ceux-ci, de préférence choisi parmi des homopolymères, copolymères et terpolymères de (méth)acrylamides d'alkyle et de quelconques mélanges de ceux-ci, plus préférablement le polymère est poly(N-isopropylacrylamide).

7. Dispositif microfluidique (10) selon l'une quelconque des revendications 1 à 6, comprenant en outre au moins une entrée (24) et au moins une sortie (26) permettant respectivement l'introduction ou le retrait de réactifs dans le dispositif (10).

8. Dispositif microfluidique (10) selon l'une quelconque des revendications 1 à 7, dans lequel le premier substrat (14) et le second substrat (20) sont indépendamment fabriqués dans un matériau choisi parmi : silicium, quartz, verre, polydiméthylsiloxane, thermoplastiques tels que copolymères oléfiniques cycliques et polycarbonates, de préférence verre et polydiméthylsiloxane.

9. Dispositif microfluidique (10) selon l'une quelconque des revendications 1 à 8, dans lequel la pluralité de motifs fermés (16) est fabriquée dans un hydrogel gonflable actionnable et le second substrat (20) est fabriqué dans un matériau non susceptible de gonfler.

10. Dispositif microfluidique (10) selon l'une quelconque des revendications 1 à 8, dans lequel le second substrat (20) est fabriqué dans l'hydrogel gonflable actionnable et les motifs fermés (16) sont fabriqués dans un matériau non susceptible de gonfler.

11. Dispositif microfluidique (10) selon l'une quelconque des revendications 1 à 10, dans lequel une pluralité de ligands est greffée sur le premier substrat (14) et/ou sur le second substrat (20).

12. Dispositif microfluidique (10) selon l'une quelconque des revendications 1 à 10, dans lequel une pluralité de ligands conjugués à un acide nucléique est associée par hybridation à au moins une partie des acides nucléiques (22) greffés.

13. Dispositif microfluidique (10) selon la revendication 11 ou 12, dans lequel chaque ligand est indépendamment choisi parmi le groupe constitué par anticorps, fragments d'anticorps, lectines et aptamères.

14. Dispositif microfluidique (10) selon l'une quelconque des revendications 1 à 13, dans lequel des acides nucléiques (22) partageant le même code-barres ont une pluralité de séquences.

15. Dispositif microfluidique (10) selon l'une quelconque des revendications 1 à 14, dans lequel des acides nucléiques (22) comprennent l'une ou de quelconques combinaisons des séquences suivantes :
1) un site de restriction ou un site photoclivable pour libération d'acide nucléique,
2) une séquence complémentaire à une amorce d'amplification pour amplification ultérieure,
3) une séquence de promoteur d'ARN polymérase T7 pour transcription in vitro (IVT),
4) un site d'hybridation, un site de ligature ou un site de recombinaison, pour marquage d'acide nucléique, et
5) une séquence de résidus nucléotidiques randomisés qui fonctionnent en tant qu'identifiant moléculaire unique (UMI).

16. Procédé de fabrication d'un dispositif (10) selon les revendications 1 à 15, ledit procédé comprenant :
a) la fourniture d'un premier substrat (14),
b) le greffage sur la surface dudit premier substrat (14) d'une pluralité de motifs fermés (16),
c) la fourniture d'un second substrat (20),
d) le greffage d'une pluralité d'acides nucléiques (22) soit sur la surface du premier substrat (14), soit sur la surface du second substrat (20), dans lequel chaque acide nucléique (22) comprend un code-barres qui encode la position de l'acide nucléique (22) sur ledit premier (14) ou second (20) substrat ;
e) le positionnement du premier substrat (14) et du second substrat (20) en plaçant les motifs fermés (16) et les acides nucléiques (22) entre le premier substrat (14) et le second substrat (20),
f) la liaison des premier (14) et second (20) substrats.

17. Procédé selon la revendication 16, comprenant en outre au moins une des étapes suivantes :
A) le greffage d'une pluralité de ligands sur la surface du premier substrat (14) et/ou sur la surface du second substrat (20), et/ou
B) l'association à la pluralité d'acides nucléiques (22) greffés d'une pluralité de ligands par hybridation, la pluralité de ligands étant conjuguée à un acide nucléique étant complémentaire à au moins une partie des acides nucléiques (22) greffés, et/ou
C) le revêtement d'un revêtement d'adhésion sur au moins une partie de la surface du premier substrat (14) et/ou du second substrat (20), et l'association audit revêtement d'adhésion d'une pluralité de ligands par liaison non covalente.

18. Procédé selon l'une quelconque des revendications 16 et 17, dans lequel ledit procédé comprend en outre une ou plusieurs des étapes suivantes :
1) hybridation d'un ADN comprenant une séquence complémentaire à la totalité ou à une partie d'une séquence constante présente dans des acides nucléiques (22) greffés ;
2) extension de l'hybridation d'ADN par polymérisation ;
3) ligature des acides nucléiques (22) greffés avec une séquence d'ADN ;
4) libération de la totalité ou d'une partie de l'acide nucléique (22) greffé, éventuellement modifié précédemment par hybridation, extension ou ligature selon 1), 2) ou 3), à partir de la surface du premier (14) ou second (20) substrat, par clivage.

19. Procédé de mise en œuvre d'analyse de cellules ou d'organites comprenant :
a) la fourniture d'un dispositif microfluidique (10) selon les revendications 1 à 15 et d'une préparation de cellules ou d'organites ;
b) facultativement, l'association de la totalité ou d'une partie des cellules ou organites avec une séquence d'acides nucléiques de marquage commune ou avec une pluralité de séquences d'acides nucléiques de marquage différentes ;
c) l'injection dans le dispositif microfluidique (10) des cellules ou organites en suspension dans des conditions dans lesquelles l'hydrogel est dans un état rétracté ;
d) la modification des conditions pour actionner l'hydrogel vers l'état gonflé, en piégeant de ce fait des cellules ou organites dans une cage formée par les première (14) et seconde (20) parois du dispositif microfluidique (10), et le motif fermé (16) d'hydrogel dans un état gonflé ;
e) facultativement l'analyse des cellules ou organites capturés et/ou des molécules qu'ils sécrètent, à l'aide d'une imagerie optique ;
f) facultativement la libération dans la cage, des acides nucléiques (22) greffés à partir de la surface du premier (14) ou second (20) substrat du dispositif microfluidique (10) ;
g) facultativement, la lyse des cellules ou organites piégés, en libérant de ce fait des acides nucléiques de cellules ou d'organites dans les cages ;
h) l'association du code-barres des acides nucléiques (22) soit avec les acides nucléiques de cellules ou d'organites libérés et/ou les séquence(s) d'acides nucléiques de marquage en formant de ce fait des acides nucléiques à code-barres ;
i) la modification des conditions pour actionner l'hydrogel vers l'état rétracté ;
j) la libération des acides nucléiques (22) greffés provenant du premier (14) ou second (20) substrat du dispositif microfluidique (10), s'ils ne sont pas libérés en f) ;
k) la récupération et le séquençage des acides nucléiques à code-barres ;
l) facultativement le mappage des données de séquençage à code-barres sur les données provenant de l'imagerie optique obtenues en e).

20. Procédé de mise en œuvre d'analyse de cellules ou d'organites selon la revendication 19, dans lequel ledit procédé comprend en outre :
e1) la liaison d'un analyte ou d'analytes sécrétés ou libérés par les cellules ou organites capturés à des ligands greffés directement ou indirectement à la surface du premier substrat (14) et/ou sur la surface du second substrat (20) ;
e2) la détection de l'analyte ou des analytes liés au ligand greffé par liaison avec un second ligand marqué ou des ligands marqués qui est/sont spécifique(s) de l'analyte ou des analytes liés.

21. Procédé de mise en œuvre d'analyse de cellules ou d'organites selon la revendication 20, dans lequel à l'étape e2), une détection est mise en œuvre
i) directement avec un second ligand ou des ligands marqué(s) par fluorescence ; ou
ii) indirectement, avec un second ligand ou des ligands marqué(s) avec un acide nucléique d'identification de ligand qui est spécifique de l'analyte ou des analytes qui est(sont) lié(s) au ligand greffé, dans lequel la séquence dudit acide nucléique d'identification de ligand permet l'identification du ligand et de l'analyte ou des analytes lié(s) au ligand greffé et s'associe au code-barres de l'acide nucléique (22), en formant de ce fait des acides nucléiques à code-barres.
